# EUROPEAN PATENT SPECIFICATION

(11) **EP 1 798 651 B1**
(45) Date of publication and mention of the grant of the patent: **17.03.2010**
(21) Application number: 06025452.1
(22) Date of filing: 08.12.2006
(51) Int. Cl.: G06F 19/00

(54) **Gene information display method and apparatus**
Vorrichtung und Verfahren zur Geninformationsanzeige
Appareil et procédé d'affichage d'informations génétiques

(30) Priority: 14.12.2005 JP 2005360528; 31.03.2006 JP 2006099742
(43) Date of publication of application: 20.06.2007
(73) Proprietor: HITACHI SOFTWARE ENGINEERING CO., LTD., Yokohama-shi, Kanagawa 230-0045 (JP)
(72) Inventor: Matsumoto, Toshiko, Shinagawa-ku Tokyo 140-0002 (JP); Nakashige, Ryo, Shinagawa-ku Tokyo 140-0002 (JP); Nozaki, Yasuyuki, Shinagawa-ku Tokyo 140-0002 (JP)
(74) Representative: Liesegang, Roland

(56) References cited:
- EP-A- 1 128 311
- WO-A-98/00708
- US-B1- 6 274 317
- PÁLSSON B ET AL: "Using quality measures to facilitate allele calling in high-throughput genotyping" GENOME RESEARCH, COLD SPRING HARBOR LABORATORY PRESS, WOODBURY, NY, US, vol. 9, no. 10, October 1999 (1999-10), pages 1002-1012, XP002408730 ISSN: 1088-9051
- MATSUMOTO T ET AL: "Novel algorithm for automated genotyping of microsatellites" NUCLEIC ACIDS RESEARCH, OXFORD UNIVERSITY PRESS, SURREY, GB, vol. 32, no. 20, 19 November 2004 (2004-11-19), pages 6069-6077, XP007901915 ISSN: 0305-1048

### SEQUENCE LISTING

<110> Hitachi Software Engineering Co., Ltd
   <120> A method and an apparatus for displaying gene information
<130> 118A002
<140>
   <141>
<150> Japan, 2005-360528
   <151> 2005-12-14
<160> 18
<170> PatentIn Ver. 2.1
<210> 1
   <211> 10
   <212> DNA
   <213> Artificial Sequence
<220>
   <223> Description of Artificial Sequence:Synthesized DNA
<400> 1
   atatatatat 10
<210> 2
   <211> 14
   <212> DNA
   <213> Artificial Sequence
<220>
   <223> Description of Artificial Sequence:Synthesized DNA
<400> 2
   atatatatat atat 14
<210> 3
   <211> 12
   <212> DNA
   <213> Artificial Sequence
<220>
   <223> Description of Artificial Sequence:Synthesized DNA
<400> 3
   atatatatat at 12
<210> 4
   <211> 12
   <212> DNA
   <213> Artificial Sequence
<220>
   <223> Description of Artificial Sequence:Synthesized DNA
<400> 4
   atatatatat at 12
<210> 5
   <211> 66
   <212> DNA
   <213> Artificial Sequence
<220>
   <223> Description of Artificial Sequence:Synthesized DNA
<400> 5
   atgcgactat gcgactgcgc ctatatatat atatatatat atatatcggc tacgagctta 60
   cgagct 66
<210> 6
   <211> 58
   <212> DNA
   <213> Artificial Sequence
<220>
   <223> Description of Artificial Sequence:Synthesized DNA
<400> 6
   atgcgactat gcgactgcgc ctatatatat atatatatcg gctacgagct tacgagct 58
<210> 7
   <211> 66
   <212> DNA
   <213> Artificial Sequence
<220>
   <223> Description of Artificial Sequence:Synthesized DNA
<400> 7
   atgcgactat gcgactgcgc ctatatatat atatatatat atatatcggc tacgagctta 60
   cgagct 66
<210> 8
   <211> 66
   <212> DNA
   <213> Artificial Sequence
<220>
   <223> Description of Artificial Sequence:Synthesized DNA
<400> 8
   atgcgactat gcgactgcgc ctatatatat atatatatat atatatcggc tacgagctta 60
   cgagct 66
<210> 9
   <211> 58
   <212> DNA
   <213> Artificial Sequence
<220>
   <223> Description of Artificial Sequence:Synthesized DNA
<400> 9
   atgcgactat gcgactgcgc ctatatatat atatatatcg gctacgagct tacgagct 58
<210> 10
   <211> 58
   <212> DNA
   <213> Artificial Sequence
<220>
   <223> Description of Artificial Sequence:Synthesized DNA
<400> 10
   atgcgactat gcgactgcgc ctatatatat atatatatcg gctacgagct tacgagct 58
<210> 11
   <211> 66
   <212> DNA
   <213> Artificial Sequence
<220>
   <223> Description of Artificial Sequence:Synthesized DNA
<400> 11
   atgcgactat gcgactgcgc ctatatatat atatatatat atatatcggc tacgagctta 60
   cgagct 66
<210> 12
   <211> 66
   <212> DNA
   <213> Artificial Sequence
<220>
   <223> Description of Artificial Sequence:Synthesized DNA
<400> 12
   atgcgactat gcgactgcgc ctatatatat atatatatat atatatcggc tacgagctta 60
   cgagct 66
<210> 13
   <211> 66
   <212> DNA
   <213> Artificial Sequence
<220>
   <223> Description of Artificial Sequence:Synthesized DNA
<400> 13
   atgcgactat gcgactgcgc ctatatatat atatatatat atatatcggc tacgagctta 60
   cgagct 66
<210> 14
   <211> 67
   <212> DNA
   <213> Artificial Sequence
<220>
   <223> Description of Artificial Sequence:Synthesized DNA
<400> 14
   atgcgactat gcgactgcgc ctatatatat atatatatat atatatcggc tacgagctta 60
   cgagcta 67
<210> 15
   <211> 64
   <212> DNA
   <213> Artificial Sequence
<220>
   <223> Description of Artificial Sequence:Synthesized DNA
<400> 15
   atgcgactat gcgactgcgc ctatatatat atatatatat atatcggcta cgagcttacg 60
   agct 64
<210> 16
   <211> 65
   <212> DNA
   <213> Artificial Sequence
<220>
   <223> Description of Artificial Sequence:Synthesized DNA
<400> 16
   atgcgactat gcgactgcgc ctatatatat atatatatat atatcggcta cgagcttacg 60
   agcta 65
<210> 17
   <211> 68
   <212> DNA
   <213> Artificial Sequence
<220>
   <223> Description of Artificial Sequence:Synthesized DNA
<400> 17
   atgcgactat gcgactgcgc ctatatatat atatatatat atatatatcg gctacgagct 60
   tacgagct 68
<210> 18
   <211> 69
   <212> DNA
   <213> Artificial Sequence
<220>
   <223> Description of Artificial Sequence:Synthesized DNA
<400> 18
   atgcgactat gcgactgcgc ctatatatat atatatatat atatatatcg gctacgagct 60
   tacgagcta                                                          69

## Description

### BACKGROUND OF THE INVENTION

### 1. Field of the Invention

The present invention relates to a method and apparatus for displaying gene information for use in the analysis of sites on a genome sequence where polymorphism between individuals is observed. Particularly, the invention relates to a method and apparatus for displaying the result of distinguishing a signal from a subject under analysis from noise signal when extracting and detecting a DNA fragment containing gene of the analyzed subject using PCR or electrophoresis, in such a manner as to be easily understandable to the user.

### 2. Background Art

The importance of automating the process of genotype determination is increasing. Now that the genome sequences of a number of living species including the human species have been completely determined, genotype determination is used for the search for genes involved in the determination of the phenotype. There is also growing demand for the identification of individuals using DNA for the purpose of criminal or terrorism investigations or as countermeasures against natural disasters or the identification of economic animals using DNA.

### Microsatellite

Normally, the genomes of living organisms of the same species have very similar base sequences, but they have different bases at several sites. For example, at one gene locus, one individual may have A while another individual may have T. This phenomenon where polymorphism is observed with respect to a single base on the genome of individuals is referred to as SNP (Single Nucleotide Polymorphism).

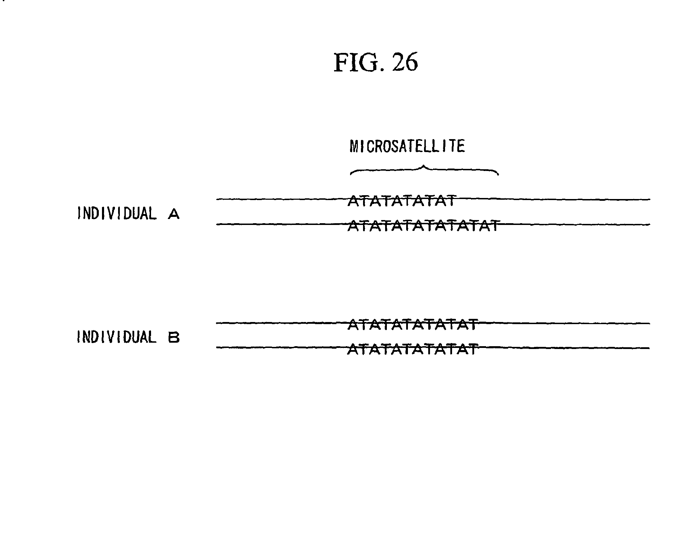

On the other hand, in the genome of living species, there are many (several tens of thousands or more) locations where a short sequence pattern of two to six bases appears repeatedly several to dozens of times. Such characteristic sequence pattern is referred to as a microsatellite. An example of the microsatellite that appears on the genome is shown in Fig. 26. The repetition of bases in a microsatellite is referred to in terms of units, and the number of bases in a unit is referred to as a unit length. For example, in the microsatellite consisting of a pattern ATATATAT... shown in Fig. 26, the unit is "AT" and the unit length corresponds to two bases. As shown in Fig. 26, the number of repetitions of a unit in a microsatellite may vary from one individual to another even when the individuals share the same unit and unit length.

As mentioned above, because SNP and the microsatellite may vary between individuals, they represent easily identifiable portions on the genome from other base sequences, and are also experimentally easily detectable. In some living species, the approximate positions of SNP and microsatellites on the genome are known, so that they can be used as genome position indicators. From these characteristics, SNP and microsatellites are referred to as DNA markers. In particular, because a microsatellite includes a plurality of bases, it contains more information than SNP and is often used as a DNA marker.

As shown in Fig. 26, each individual of many living species carries a pair of genomes (homologous chromosomes) deriving from the female gamete and the male gamete. The genes that exist at corresponding sites on the pair of genomes are called alleles, and such a combination of alleles is referred to as a genotype. As mentioned above, SNP or microsatellites are associated with portions on the genome where the base sequence may vary from one individual to another. Thus, generally two (or three or more, in rare occasions) alleles are associated with SNP, and several to 20 or more kinds of alleles are present in a microsatellite.

In the example shown in Fig. 26, individual A carries two alleles in which the unit "AT" is repeated five times in one allele and seven times in the other allele, while individual B carries two alleles in each of which the unit "AT" is repeated six times. The state in which individual alleles of different kinds are carried by an individual, such as individual A, is referred to as heterozygous. On the other hand, the state in which the two alleles of the same kind are carried by an individual, such as individual B, is referred to as the homozygous.

### PCR and electrophoresis experiment

When a microsatellite is used as a DNA marker, an experiment involving PCR (Polymerase Chain Reaction) or electrophoresis is conducted so as to extract and detect a site on the genome where the microsatellite is present. PCR is an experiment technique whereby a pair of base sequences referred to as primer sequences at both ends of a microsatellite are designated, and only the portion between them in the microsatellite is repeatedly duplicated as a DNA fragment so as to obtain a certain quantity of sample. Electrophoresis, which includes gel electrophoresis and capillary electrophoresis, is an experiment technique whereby an amplified DNA fragment is caused to migrate in a charged migration path so as to separate DNA fragments with different lengths. It is a sample separation technique that takes advantage of the difference in migration rate in the migration path depending on the length of the DNA fragment (the longer the DNA fragment, the smaller its migration rate).

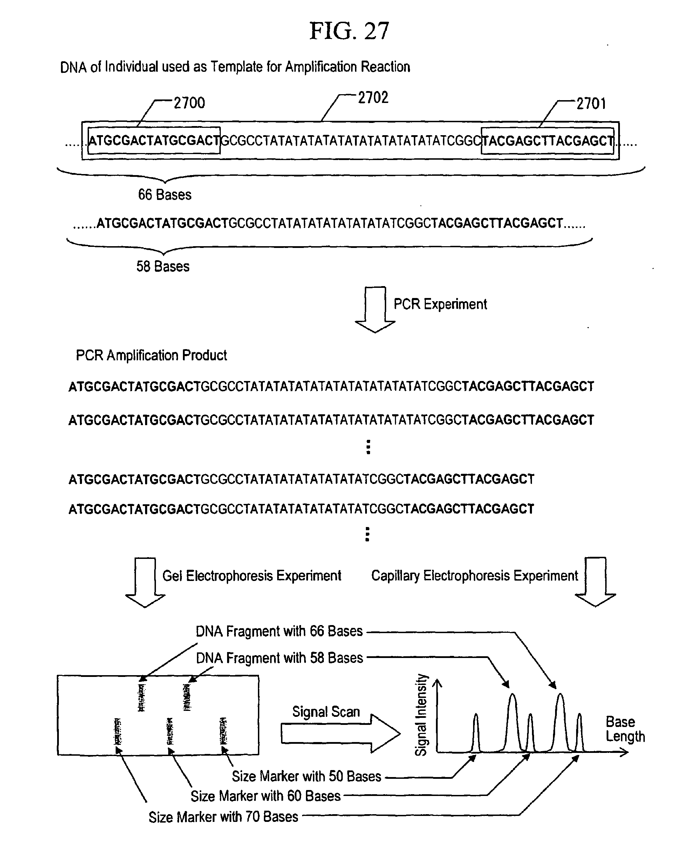

Fig. 27 schematically shows an experiment procedure for extracting and amplifying a DNA fragment in a microsatellite portion by PCR and gel electrophoresis. First, a pair of primer sequences 2700 and 2701 with a microsatellite of interest located between them are designated, and then a genome region 2702 containing the microsatellite and the primer sequences is amplified by an PCR experiment. In the example shown in Fig. 27, the numbers of repetitions in the microsatellites on the two homologous chromosomes are different; namely, the example is heterozygous. Because the lengths of the individual microsatellite portions are different, two types of PCR amplification products, i.e., DNA fragments having different lengths (one with 66 bases and the other 58 bases) are obtained. When these are subjected to electrophoresis on a gel plate for a certain time, the aforementioned two kinds of PCR amplification products are separated from each other based on the difference in the length of the DNA fragments. Each DNA fragment is labeled with fluorescence dye, and after electrophoresis, the intensity and position of a fluorescence signal from each DNA fragment are detected. Consequently, a graph is obtained in which, as shown in Fig. 27, the length of the DNA fragment (i.e., the distance migrated) is shown on the horizontal axis, and the intensity of fluorescence signal (i.e., the quantity of the DNA fragments present) is shown on the vertical axis. Furthermore, by applying a DNA fragment with a known length (also referred to as a size marker) to electrophoresis in addition to the PCR amplification products, and detecting a fluorescence signal therefrom, the length of each PCR amplification product can be determined with respect to the detected position of the size marker.

While experiment methods using gel electrophoresis have been described above, the same procedure can be carried out using capillary electrophoresis. In capillary electrophoresis, a sample is caused to migrate through a thin tube filled with a gel, and the time each type of sample takes to migrate a certain distance (normally to the end of the capillary) is measured, thereby examining the length of the DNA fragment. In capillary electrophoresis, generally a fluorescence signal from the sample is detected with a fluorescence signal detector installed at the end of the capillary, instead of detecting a fluorescence signal from the sample in the gel by scanning.

### Noise produced in the PCR and electrophoresis experiments

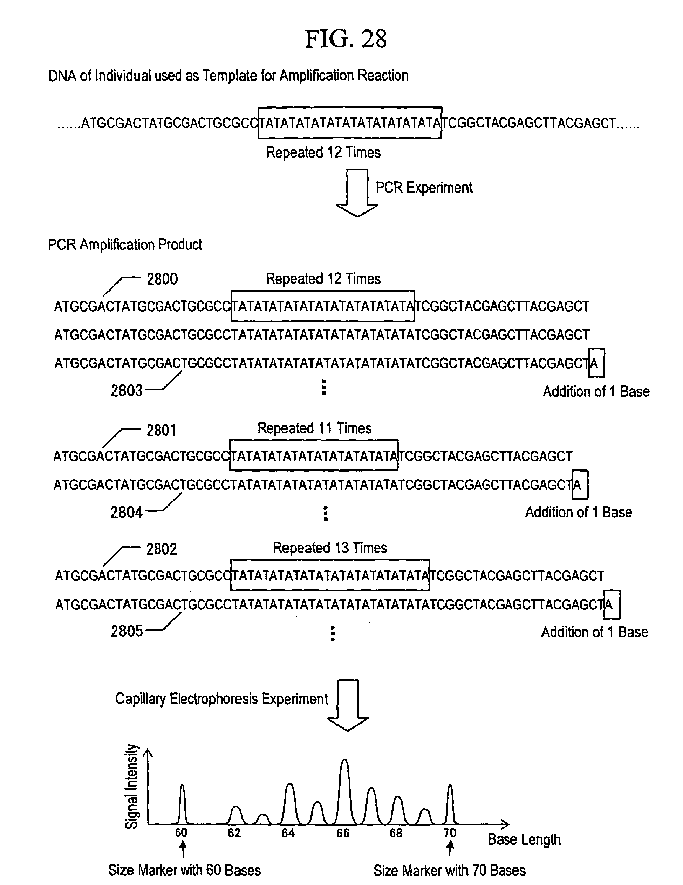

The experimental results shown in Fig. 27 above are obtained when the PCR and electrophoresis are carried out in an ideal process. In actual experiments, various kinds of noise may be produced. In the following, a stutter peak and a +A peak, which are typical noise produced in the PCR and electrophoresis experiment processes, will be described with reference to Fig. 28. For simplicity's sake, Fig. 28 shows only the DNA fragment with 66 bases (which includes the microsatellite in which "TA" is repeated 12 times) from those shown in Fig. 27.

The stutter peak is a noise caused by a phenomenon wherein the number of repetitions in a microsatellite portion in the DNA fragment as the subject for duplication increases or decreases due to slipped-strand mispairing upon PCR reaction. Specifically, the DNA fragment in which the number of repetition has increased or decreased is observed as a noise peak during fluorescence analysis. As shown in Fig. 28, aside from the DNA fragment 2800 that includes a normal microsatellite in which the unit "TA" is repeated 12 times, DNA fragment 2801 or 2802 is produced that includes an abnormal microsatellite in which the unit "TA" is repeated 11 or 13 times, which is observed as a stutter peak during fluorescence analysis. Because an increase or decrease with a greater number of repetition could be caused, it is possible that by performing PCR, a DNA fragment is produced of which the length has increased or decreased by the integer multiple of the unit length of the microsatellite, in addition to the DNA fragment with the same length as the original DNA fragment (66 bases).

The +A peak is a noise caused by a phenomenon in which, when duplicating a DNA fragment by PCR, an excess base (A, normally) is added to the DNA fragment. Specifically, a DNA fragment to which one base is added is observed as a noise peak in fluorescence analysis. As shown in Fig. 28, in addition to a DNA fragment 2803 that is produced in which one base is added to the normally duplicated DNA fragment 2800, DNA fragments 2804 and 2805 could be produced in which one base is added to abnormal DNA fragments 2801 and 2802 in which the number of repetition in the microsatellite portion has increased or decreased by slipped-strand mispairing. Such DNA fragments 2803, 2804, and 2805 are observed in the form of different +A peaks during fluorescence analysis.

In the graph of Fig. 28 showing the results of fluorescence analysis, the DNA fragment having 66 bases, which is the same length as the original DNA fragment, is the peak that should be observed (to be hereafter referred to as a "true peak"). The other peaks are all noise peaks. It is seen that stutter peaks appear at intervals corresponding to the unit length of the microsatellite with respect to the true peak (i.e., at positions of 62 bases, 64 bases, and 68 bases). It is also seen that +A peaks appear at positions that are longer than those of the true peaks or the stutter peaks by one base (i.e., at positions of 63 bases, 65 bases, 67 bases, and 69 bases). Namely, the +A peaks that appear at positions 63 bases, 65 bases, 67 bases, and 69 bases correspond to the DNA fragments of the base length of 62 bases, 64 bases, 66 bases, and 68 bases, respectively, to which one base has been added. In the following, a true peak or a stutter peak that corresponds to a DNA fragment to which the one base is not added and that is responsible for a particular +A peak is referred to as "an original peak."

In the microsatellites on a set of genomes, there are homozygotes and heterozygotes, and the graph waveform of the fluorescence signal varies greatly depending on whether the extracted DNA fragment is a homozygote or a heterozygote. If it is a homozygote, just one true peak would appear in the graph. If it is a heterozygote, two true peaks would appear in the graph. However, as will be seen from the graph of Fig. 28 showing the results of fluorescence analysis, there are cases where a number of peaks appear even in the case of a homozygote. Thus, it is not easy to determine whether the extracted DNA fragment is a homozygote or a heterozygote based on the graph waveform of the fluorescence signal or the number of peaks. Furthermore, in the case of a heterozygote, peaks may overlap or the height of two true peaks may not be exactly the same, resulting in a very complex waveform.

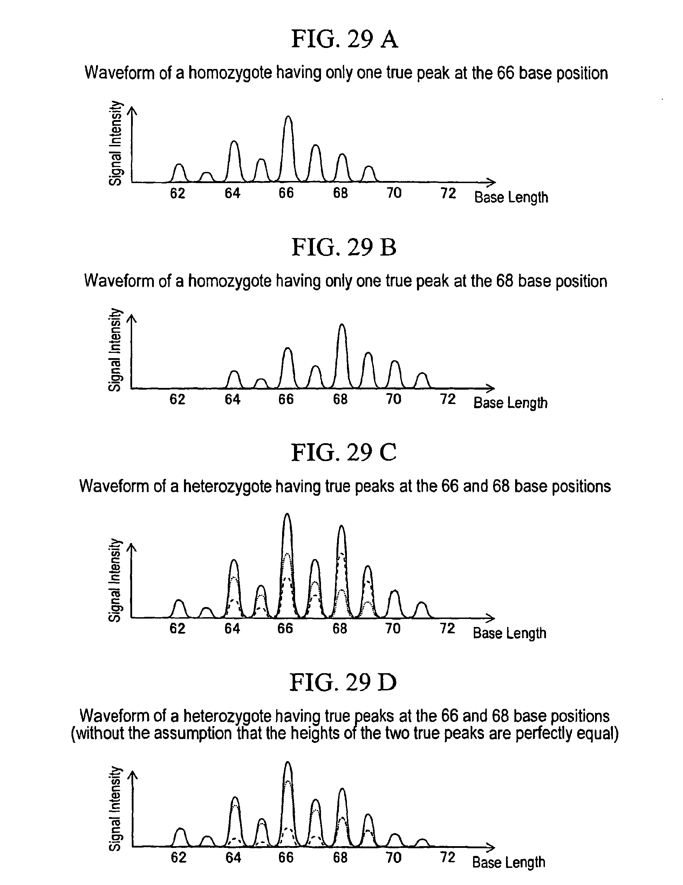

In the following, waveforms that appear in homozygotes and heterozygotes will be described with reference to Fig. 29. First, waveforms that appear in the case of homozygotes are shown in Fig. 29(a) and (b). Fig. 29(a) shows a waveform obtained from a DNA fragment with 66 bases, while (b) shows a waveform obtained from a DNA fragment with 68 bases. Next, waveforms that appear in the case of heterozygotes are shown in Fig. 29(c) and (d). It is assumed herein that a DNA fragment with 66 base and a DNA fragment with 68 bases are obtained as PCR amplification products from each heterozygote (Figs. 29(c) and (d)). Fig. 29(c) shows a waveform obtained by composing waveforms from these DNA fragments (one indicated by a dotted line and the other by a dashed line), assuming that the height of the true peak due to the DNA fragment with 66 bases in the PCR products is equal to that of the true peak due to the DNA fragment with 68 bases. Fig. 29(d) shows a composed waveform in a case where the true peak due to the DNA fragment with 66 bases and the true peak due to the DNA fragment with 68 bases, both of which are included in the PCR amplification product, have different heights.

In the experimental process of PCR and electrophoresis, it is important to distinguish a true peak from other noise peaks from among a plurality of peaks observed during fluorescence analysis. Known software for detecting and removing noise peaks include those disclosed in Patent Document 1, Non-patent Documents 1 and 2, TrueAllele from Cybergenetics Co., SAGA from LI-COR, Inc., GenoTyper from Applied Biosystems, and GeneMapper from Applied Biosystems.

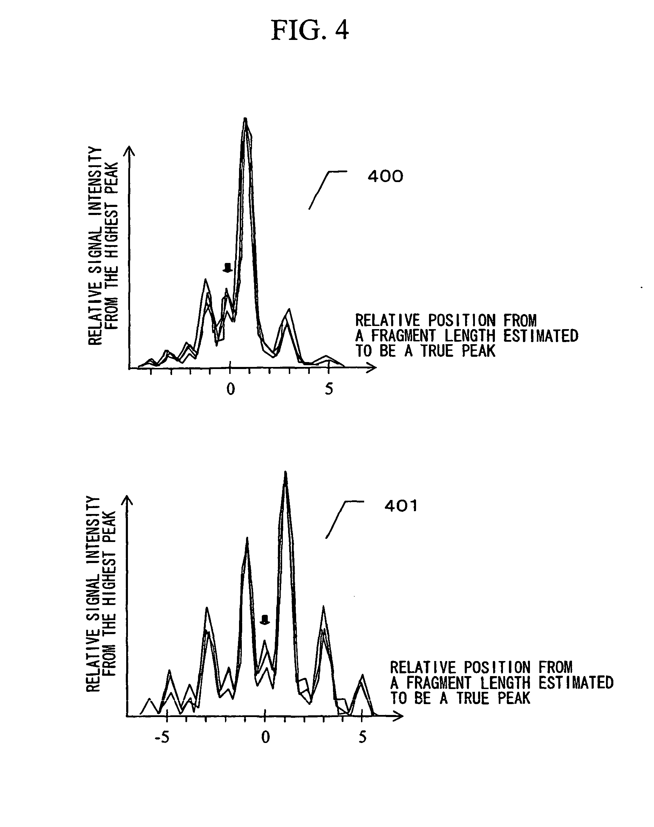

In Non-patent Document 2, a true peak determination is made based on the difference between an observed waveform and an expected waveform. This method allows for the determination of whether or not each peak is a true peak, a stutter peak, or a +A peak, as summarized below. It is known that there is a regularity in the way a stutter peak and a +A peak appear depending on each marker. Thus, as described in the section "Selection of individuals," heterozygotes in which two true peaks are sufficiently separated (i.e., obvious heterozygotes having two lumps of peak sets in the waveform), and obvious homozygotes, of both of which interpretation of the waveform is possible even if the way a stutter peak or a +A peak appears is unknown, are collected from the individuals as subjects of analysis. Then, as described in the section "Determination of stutter patterns and ratio range for +A peaks," the way the stutter peak and the +A peak appear in the marker as a subject of analysis is examined, using such individuals. In this way, it becomes possible to estimate the waveform (the stutter peak and the +A peak expected to appear in the vicinity of the true peak, and their heights) that is expected when a true peak is assumed. This example is shown in Figure 4, where a solid line shows the observed waveform and the triangle shows the waveform that is expected when a peak Pmax is assumed to be a true peak. Namely, the waveforms show, from left to right: a stutter peak shorter by 2 units; a +A peak of the stutter peak shorter by 2 units; a stutter peak shorter by 1 unit; a +A peak of the stutter peak shorter by 1 unit; a true peak; and a +A peak of the true peak. In the example shown in Figure 4, there is a large difference between the waveform observed and the expected waveform at the peak Pmax'. Therefore, the second expected waveform is calculated on the assumption that the peak Pmax' is the true peak, and the overlapping of the two expected waveforms is compared with the observed waveform. Through the foregoing processes, the following are made possible:
■ Determination as to whether each peak is a true peak, a stutter peak, or a +A peak.
■ Calculation of the ratio of the height of one true peak to that of the other true peak.
■ Calculation of the ratio of derivation of each peak in the waveform from the first or the second true peak.
■ Calculation of the waveform expected based on the estimated true peak.

Patent Document 1: U.S. Patent No. 6,274,317
Non-patent Document 1: B. Palsson et al., "Using Quality Measures to Facilitate Allele Calling in High-Throughput Genotyping", Genome Research 9 (1999), pp.1002-1012
Non-patent Document 2: T. Matsumoto et al., "Novel algorithm for automated genotyping of microsatellites", Nucleic Acids Research, Vol.32 No.20 (2004), pp.6069-6077

EP 1 128 311 discloses a method and system for DNA analysis. The method comprises the steps of forming electrophoretic data of DNA samples with DNA ladders, comparing these data, transforming the coordinates of the DNA sample's data into DNA length coordinates and analyzing the DNA sample in length coordinates.

### SUMMARY OF THE INVENTION

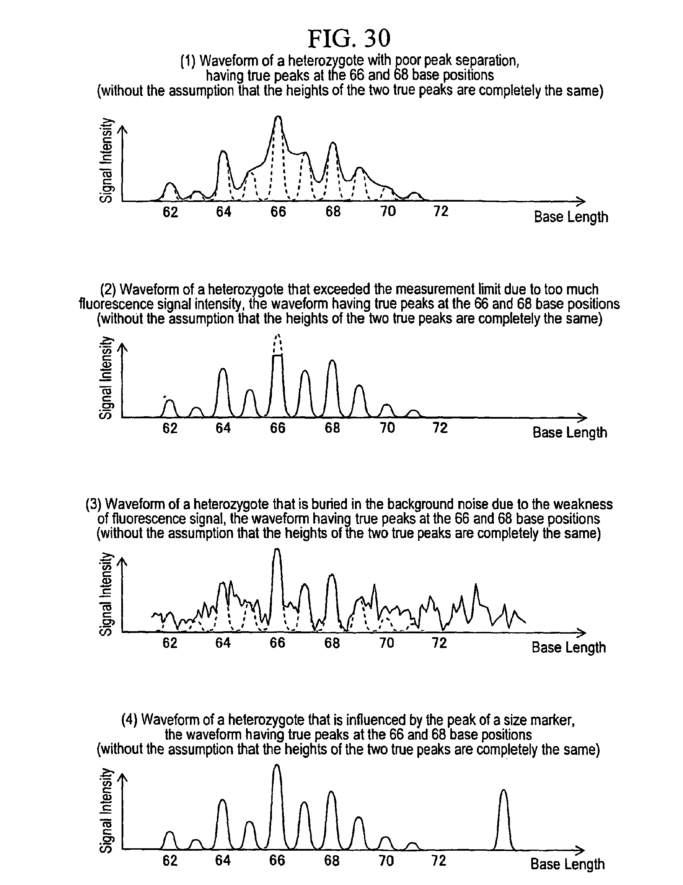

Conventionally, the result of genotype determination that is automatically carried out using a microsatellite is visually inspected by the experimenter based on a collation between relevant waveforms and the result of automatic determination. If it is judged that the experiment has not gone well, the experiment is conducted again. Such visual inspection by the experimenter poses a bottle neck in the analysis process. Particularly, in actual experiments, noise peaks do not always appear as regularly as shown in Fig. 29, and in many
cases, more complicated noise peaks appear due to the following reasons. Fig. 30 shows
graphs of waveforms obtained in experiments in which various errors were contained. The waveforms were obtained by subjecting the same PCR amplification product as in Fig. 29(d) to electrophoresis.

Fig. 30(1) shows the waveform in a case where the peaks were not completely separated. In this figure, the dashed line shows the waveform that would have resulted had the peaks been completely separated, the waveform being identical to the waveform shown in Fig. 29(d). In electrophoresis, PCR amplification products with different fragment lengths are separated using the fact that the migration rate of the PCR amplification product varies depending on the length of the fragment. The waveform shown is obtained if such separation fails. In this case, at the locations where a high peak and a low peak are adjacent to each other, the lower peak is hidden in the shadow of the higher peak and not observable. For example, a peak of 65 bases is hidden in the shadow of a peak of 66 bases, and a peak of 70 bases is hidden in the shadow of a peak of 69 bases, so that they are both unobservable.

Fig. 30(2) shows a waveform obtained in a case where the fluorescence signal was so strong as to exceed the measurement limit. In the figure, the dashed line shows the waveform obtained when the fluorescence signal was appropriately measured, which is identical to the waveform shown in Fig. 29(d). The vertical axis of the waveform graph shows the intensity of the fluorescence signal observed by the detector. In this waveform graph, the top of the peak with 66 bases in the waveform data is lacking because the fluorescence signal from the fragment with 66 bases exceeded the measurement limit of the detector. The fluorescence signal exceeds the measurement limit of the detector when, for example, too many fragments with a particular base length are included in the sample.

Fig. 30(3) shows the waveform obtained when the fluorescence signal was so weak as to be buried in the background noise. In the figure, the dashed line shows the waveform that would have resulted had the fluorescence signal been properly measured, the waveform being identical to the waveform shown in Fig. 29(d). In this waveform graph, lower peaks, such as those with 70 bases, are buried in background noise. The fluorescence signal becomes too weak when, for example, too little DNA fragments are included in the sample, as opposed to the aforementioned case (2).

Fig. 30(4) shows the waveform influenced by the peak of a size marker. Normally, a DNA fragment as a size marker is labeled with a fluorescence dye having a different color from that of a microsatellite DNA fragment, so that the two do not become mixed. However, there are cases where the peak of the size marker bleeds out onto the waveform of the microsatellite due to an error in the calculation for extracting from the detector raw data a single fluorescence dye component.

Non-patent Document 1, the software TrueAllele from Cybergenetics Co., and the software GenoMapper from Applied Biosystems provide the function for evaluating the result of automatic determination by focusing attention on peak characteristics. However, as shown in Figs. 29 and 30, various errors are made during experiments, and none of these techniques are capable of reliably and completely detecting "individuals that have been erroneously determined," making visual confirmation indispensable. Particularly, in the case of examination of individuals based on DNA for crime or terror prevention purposes, the result of determination may influence the outcome of criminal trials. Therefore, it is indispensable for the experimenter to perform visual inspection of all of the determination results. Furthermore, in order to determine the necessity of carrying out the experiment again based on visual inspection of the waveform of which the accuracy of automatic determination has been determined to be low, it is necessary to examine the tendency of the waveform (the way a stutter peak or +A peak appears, or the position where the stutter peak appears) with respect to a relevant marker. For this reason, all or most of the individuals need to be visually confirmed after all. However, the visual confirmation of the waveform results not only in greater cost as the number of markers or individuals increases, but could lead to human error.

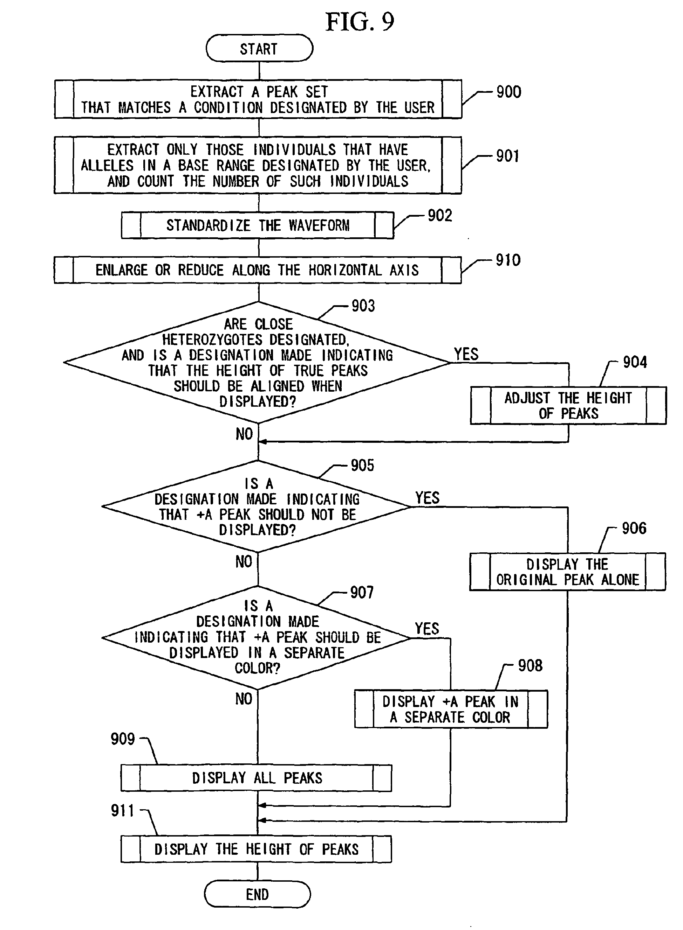

As a method for detecting individuals that include complicated noise peaks as shown in Fig. 30, a method could be used whereby waveforms are displayed in an overlapped manner and an individual is detected that has a waveform that is separated from the other waveforms. This method is often used in other fields for information processing purposes. In the field of microsatellite marker analysis, the display of a number of waveforms of individuals in an overlapped manner is referred to as an allelic ladder, which is used for examining at which position a peak can possibly appear. However, the allelic ladder cannot be used for the aforementioned purposes, i.e., to detect an individual that contains complicated noise peaks and for which the experiment has not gone well, and to detect an individual of which the result of automatic determination is erroneous. This is because the waveforms obtained from a microsatellite marker are so varying, as described above, that they cannot be simply overlapped to make sense. Figure 9 of Non-patent Document 1 shows a screen of an allelic ladder where the display screen is crowded with so many peaks that it is obviously impossible to detect on this screen an individual that has a waveform that is separated from the other waveforms.

In view of the aforementioned problems, it is an object of the present invention to provide a method and apparatus for displaying gene information, whereby waveform data, which is obtained as a result of fluorescence analysis of a DNA fragment following PCR amplification and electrophoresis, can be displayed in such a manner that an experimental error can be easily detected by visual inspection.

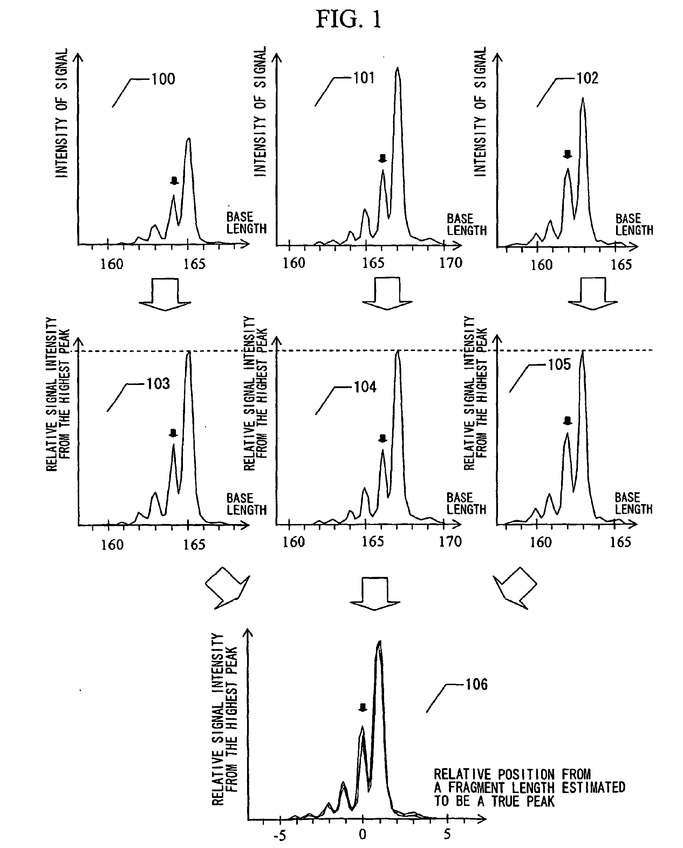

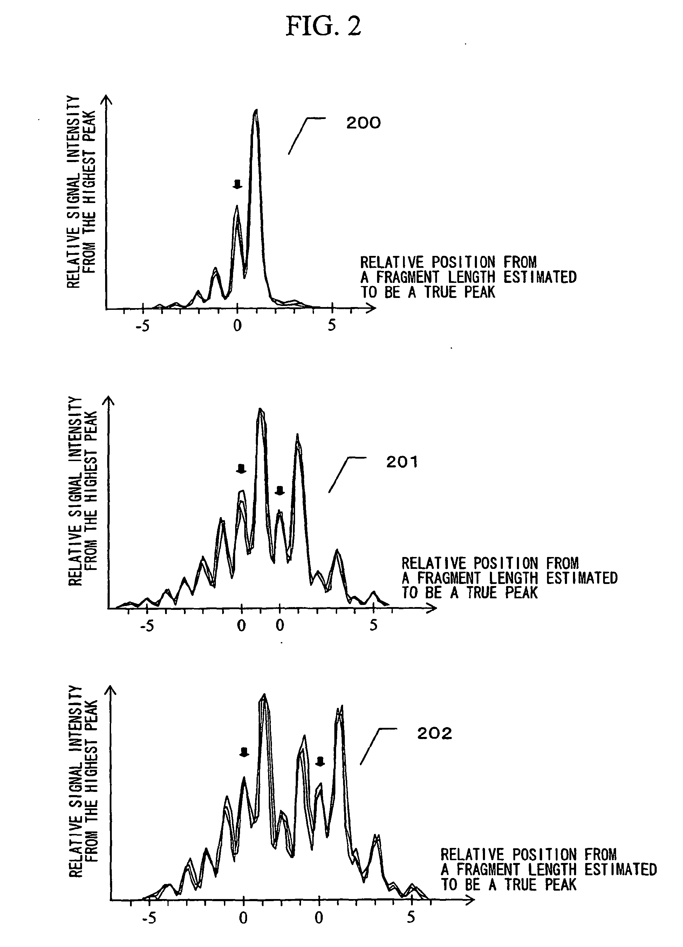

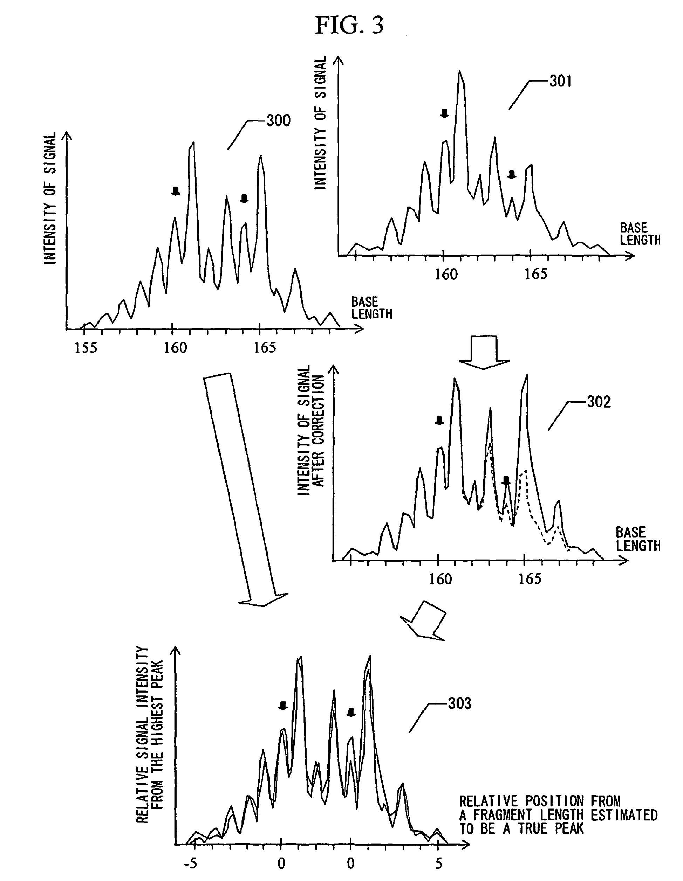

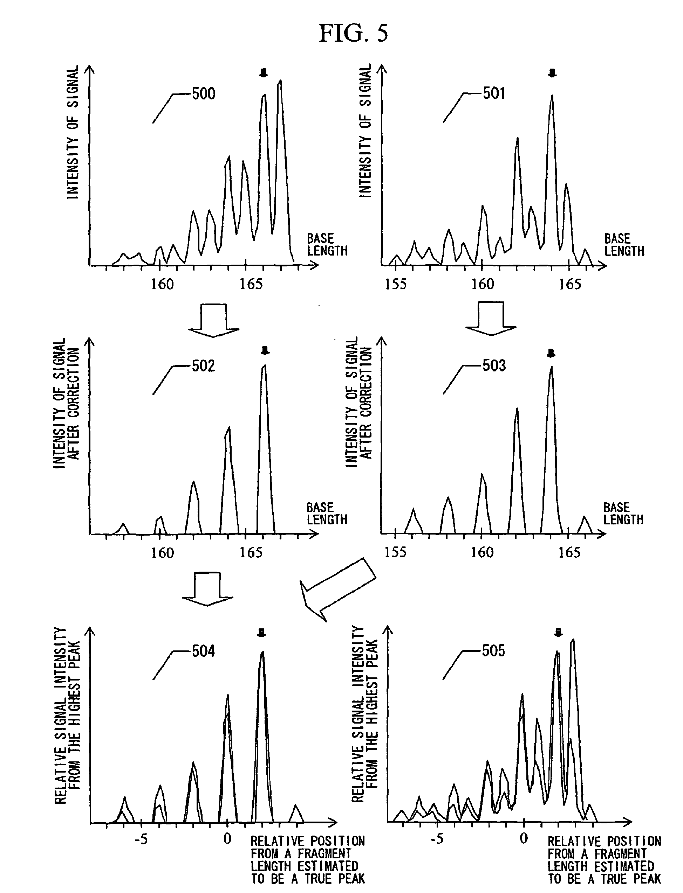

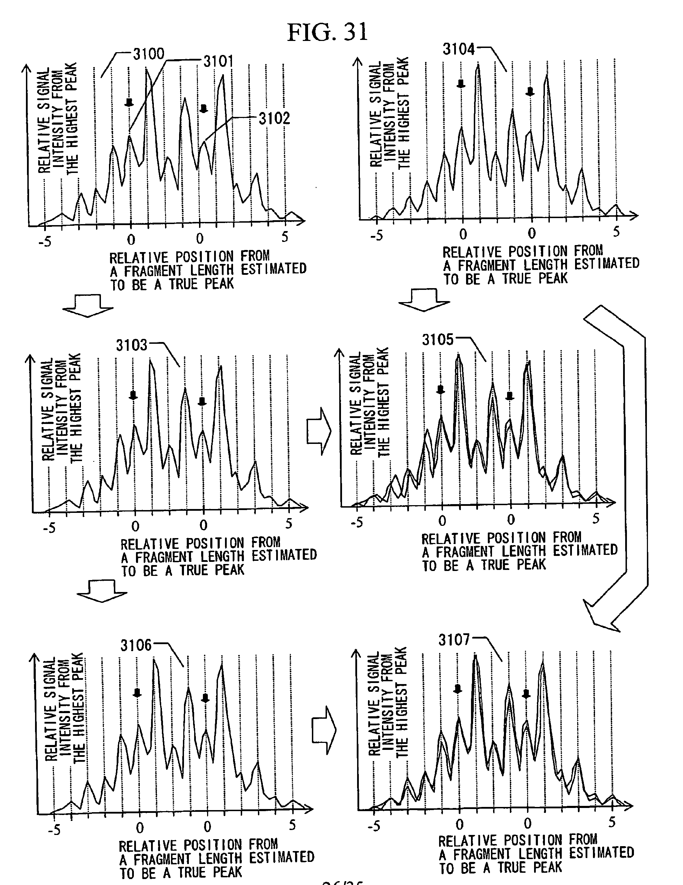

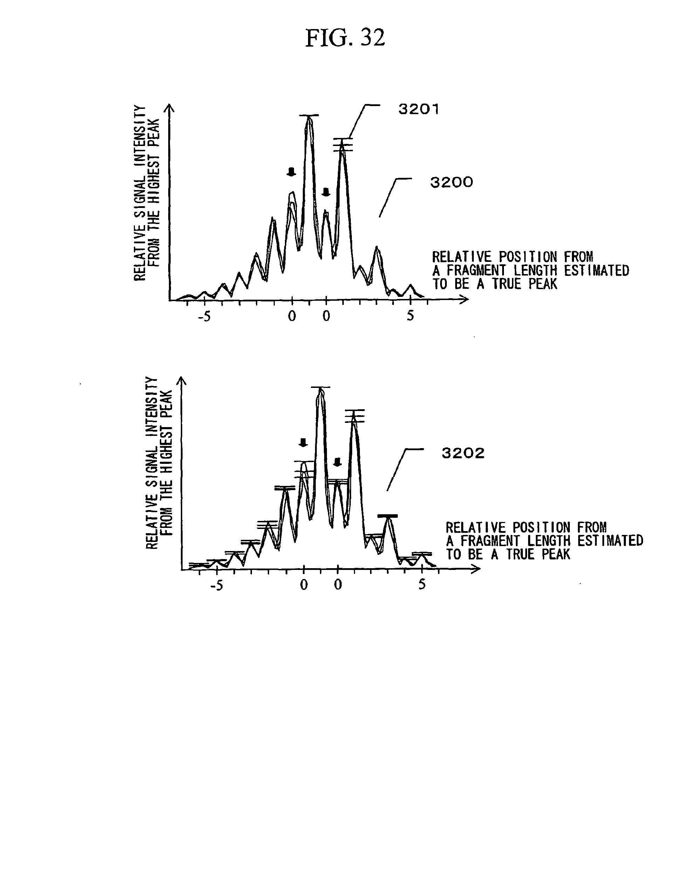

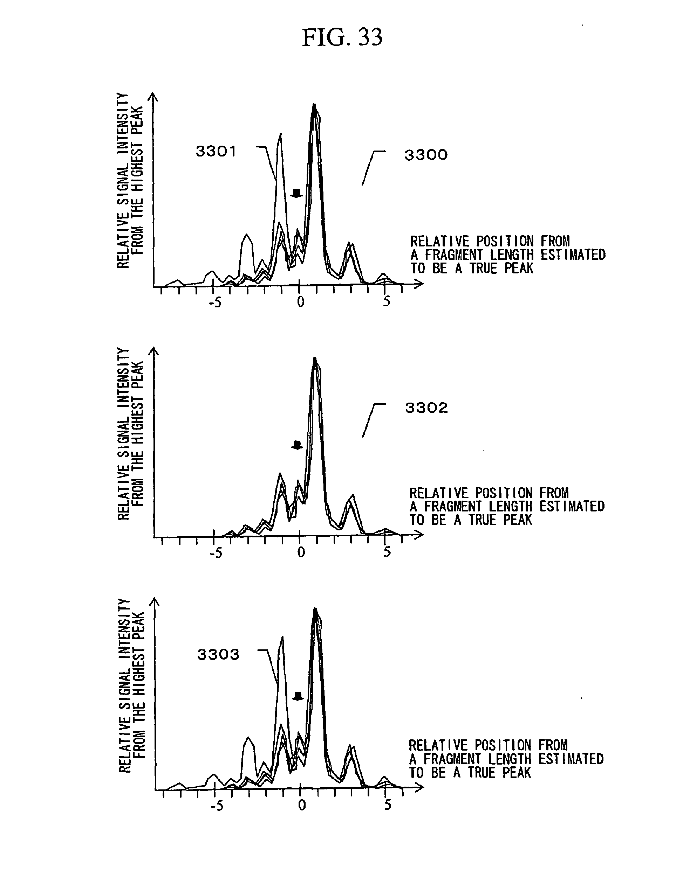

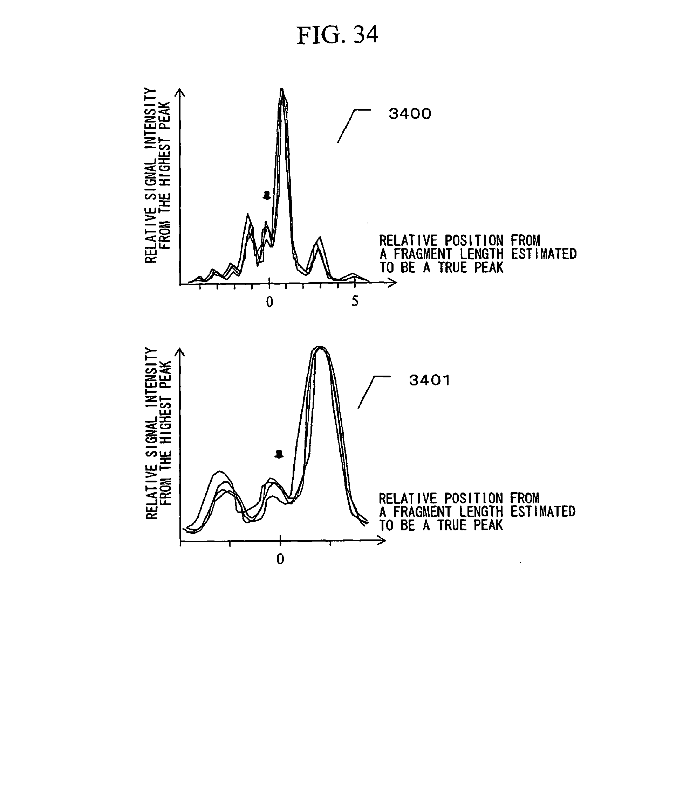

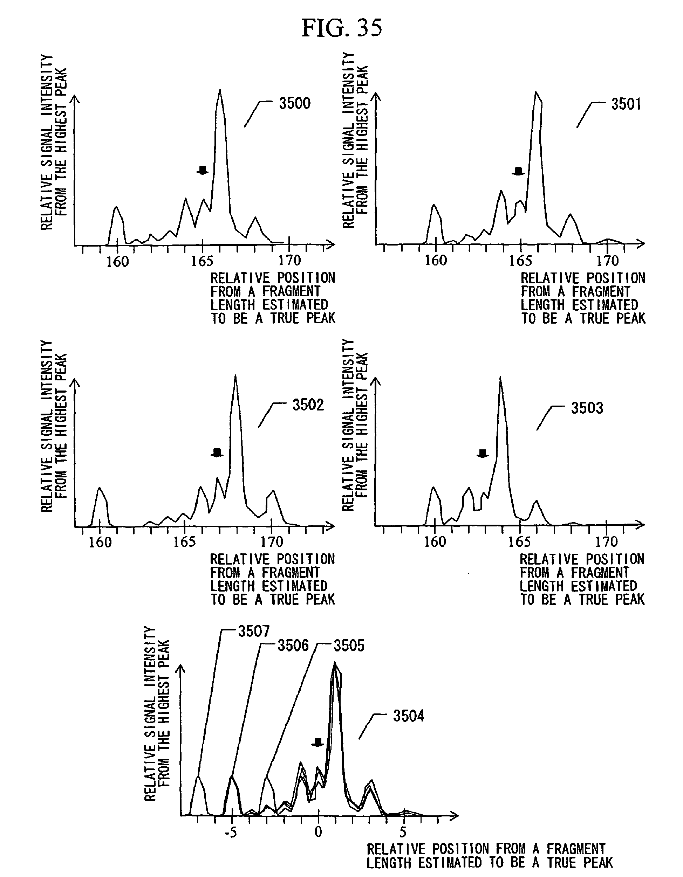

In order to achieve the aforementioned object, the invention provides for performing waveform correction and overlapped-display processes, using the below listed functions, on the waveform data obtained as a result of fluorescence analysis of a DNA fragment following PCR amplification and electrophoresis and on the result of automatic determination of the data, thereby allowing the experimenter to carry out visual inspection efficiently.
Function 1: A function for displaying the waveforms of a plurality of individuals in an overlapped manner and enabling the user to determine at once whether or not the result of automatic determination is appropriate and whether or not there are individuals for which a re-experiment should be performed. When the individual waveforms are aligned and displayed using the below-indicated functions 2, 3, 4, 5, 6, 8, 9, 10, 11, and 12, similar waveforms should be displayed for the plurality of individuals as long as there is no problem either in the result of experiment or the result of determination. By examining only an individual whose waveform is different from those of the other individuals separately and in detail, the number of steps for visual inspection can be reduced.
Function 2: A function based on Function 1 for standardizing the vertical axis and the horizontal axis of the graph that is displayed. With regard to the vertical axis, the vertical axis of each waveform is multiplied by a constant number so that the heights of the highest peaks in the waveforms are equalized. Referring to the three waveforms 100, 101, and 102 shown in Fig. 1, the highest peaks are observed at the 165 base, the 167 base, and the 163 base positions, respectively. The heights of the waveforms are multiplied by a constant number so as to equalize the heights of the peaks thereof, thereby obtaining waveforms 103, 104, and 105. With regard to the horizontal axis, the individual waveforms are translated so that the positions of the peaks that have been determined to be true peaks in each waveform are aligned. In the three waveforms 103, 104, and 105 with the thus equalized heights of the peaks, if it is determined that the peaks indicated by the arrows (the peaks observed at the 164 base, the 166 base, and the 162 base positions) are true peaks, the waveforms are translated such that these three peaks are aligned, thereby obtaining an overlapped waveform 106.
Function 3: A function based on Function 1 for displaying a plurality of individuals in an overlapped manner in accordance with the relative relationship between the alleles. Specifically, as shown in Fig. 2, the following graphs are displayed: a waveform graph 200 in which only the waveforms of homozygotes and the waveforms near one of the alleles in sufficiently separated heterozygotes are overlapped; a waveform graph 201 in which only those heterozygous individuals in which the two true peaks are separated by 1 unit are overlapped; and a waveform graph 202 in which only those heterozygous individuals in which two true peaks are separated by 2 units are overlapped. The same is true with those heterozygous individuals in which two true peaks are separated by 3 units or more. In this way, waveforms can be compared with each other under identical conditions even though noise peaks may be overlapped upon each other in heterozygous waveforms. Alternatively, the user may designate a threshold that should be permitted as an error when determining an equal relative relationship (e.g., when an error of 0.5 base should be permitted, two true peaks in a marker with the unit length of 2 bases would be determined to be separated by 2 units within threshold values of 2x2-0.5=3.5 bases or more and 2x2+0.5=4.5 bases or less). In this way, it becomes possible to collect individuals having alleles with a designated relative relationship flexibly depending on the experimental environment.
Function 4: A function based on Function 1 for performing height correction on the waveforms of heterozygotes in which the heights of the two true peaks are different. Fig. 3 shows waveforms 300 and 301 of heterozygotes that are separated by 2 units (where true peaks are observed at the 160 base and the 164 base positions). In the waveform 300, the two true peaks have almost the same height. On the other hand, in the waveform 301, the two true peaks have different heights. Thus, a peak height correction is performed on the waveform 301 so as to equalize the heights of the two true peaks, thereby obtaining a waveform 302 (where the dashed line shows the waveform prior to correction). Thereafter, the waveform 300 and the thus corrected waveform 302 are overlapped upon each other, obtaining a waveform 303.
Function 5: A function based on Function 1 whereby only the waveforms of those individuals of which the true peaks are included in a preset range of fragment length are displayed in an overlapped manner. It is known that the slipped-strand mispairing, which is responsible for the stutter peak, among other noise peaks, tends to occur more frequently as the number of repetition prior to amplification increases. Therefore, in those markers in which the difference in the number of repetition is very large between individuals, the stutter peak may appear in greatly varying ways depending on the allele. Fig. 4 shows a waveform 400 of a homozygote with the number of repetitions of less than 15, and a waveform 401 of a homozygote with the number of repetitions of 15 or more, for example. It can be seen that the stutter appears more frequently in the waveform with the number of repetitions of 15 or more. Thus, for such markers, by setting a range of fragment length in advance and overlapping the waveforms of only those individuals that include true peaks in the same range, the manner of appearance of the stutter can be unified when comparing the waveforms.
Function 6: A function based on Function 1 for eliminating +A peak from display. Of the noise peaks, the manner of appearance of +A peak has a certain tendency for each marker. For example, in the case of one marker, hardly any +A peak may be observed in any individual, and in case of another, the +A peak may be very much higher than an original peak in any individual. However, the manner of appearance of the +A peak for each individual may fluctuate somewhat. In the example shown in Fig. 5, the true peak (at the 166 base) in the waveform 500 of a certain individual is almost equal to the +A peak (at the 167 base) thereof in height. However, the height of the true peak (the 164 base) in a waveform 501 of another individual is nearly double that of the +A peak (the 165 base) thereof. For such markers, waveforms 502 and 503 are generated by eliminating the +A peak from the waveforms 500 and 501 of each individual, and they are then overlapped upon each other, producing an overlapped waveform 504. In this way, it becomes possible to compare the individual waveforms without being influenced by the variation in the way the +A appears in the individuals. Furthermore, the +A peak portion may be displayed in a separate color or with a separate type of line, as in a waveform 505. Conversely, for those markers in which the height of a +A peak is higher than that of a true peak, the true peak may be eliminated from display or shown in a separate color or with a separate type of line. Further alternatively, the stutter peak, the +A peak, and other noise peaks (several kinds of noise peaks, such as a bleed through peak, are defined by TrueAllele, which is an existing technology) may be allocated different colors or types of line, for example.
Function 7: A function for displaying an observed waveform and an expected waveform in an overlapped manner concerning the result of an experiment for a single individual. For the calculation of an expected waveform, reference can be made to Non-patent Document 2, as mentioned in the Background Art section. If the two waveforms are greatly separated from each other, this shows, visually, that the reliability of the result of automatic determination is low, thus allowing the user to make a visual inspection easily.
Function 8: A function based on Function 1 for aligning the interval of two true peaks in heterozygotes by enlarging or reducing the size of their waveforms along the horizontal axis in a region between the two true peaks of the heterozygotes. When calculating the fragment length from a size marker, an error may be introduced. If this happens, as indicated by 3100 in Fig. 31, the interval of two true peaks in an heterozygous individual assumes a value other than an integer number (i.e., if the left true peak is translated to the position of zero on the horizontal axis (3101), the right true peak is misaligned as indicated by 3102). Thus, as indicated by 3103, the waveform between the left and right true peaks is enlarged or reduced in size so that the interval between the left and right true peaks becomes exactly 4 bases. After performing this process, the waveform is overlapped on a waveform 3104 of another individual, whereby, as indicated by 3105, the waveforms can be compared with each other without being influenced by the error introduced during the calculation of the fragment length. Further, as indicated in 3106, the waveform may be enlarged or reduced in size along the horizontal axis in a region outside the true peak, so that the intervals between a true peak and a noise peak and between noise peaks can be aligned when displayed. In this way, as indicated by 3107, the waveforms can be compared with each other in the entire region of the waveform without being influenced by the error introduced during the calculation of the fragment length. When the intervals between a true peak and a noise peak and between noise peaks are aligned when the waveforms are displayed, not just heterozygotes but all of the individuals would be the subject of investigation.
Function 9: A function based on Function 1 for adding the display of height for two true peaks in heterozygotes. For those waveforms of heterozygotes in which the height of two true peaks are different, it is necessary to determine if the individual is in fact a homozygote if the height difference is extreme. As indicated by 3200 in Fig. 32, the height of two true peaks (i.e., the +A peaks of the two true peaks, since the +A peak is a higher marker than the true peak in the example shown in Fig. 32) are explicitly shown (3201). In this way, it becomes possible for the user to easily see the height of each peak in each waveform even when a number of waveforms are overlapped (e.g., whether or not there is a waveform in which the height difference between two true peaks is extreme). Further, as indicated by 3202, an indication of height of a noise peak may be added. When the display is made while correcting the peak height, as mentioned with reference to Function 4, the height prior to correction may be displayed. Furthermore, the height of each peak may be displayed in a graph such as a histogram or a boxplot, for example.
Function 10: A function based on Function 1 for displaying waveforms in an overlapped manner except for the individuals designated by the user. As indicated by 3300 in Fig. 33, it is assumed that there is an individual 3301 having a waveform different from those of the other individuals, and that the user's detailed analysis has revealed that the individual is an error caused during the genotype determination process. In this case, as indicated by 3302, the user can easily confirm the display from which the individual determined to be an error has been removed to see if there are still other individuals that require a detailed confirmation. Furthermore, as indicated by 3303, the individual determined to be an error may be displayed in a separate color or with a separate type of line. Further alternatively, individuals other than those designated by the user, individuals that have been evaluated, as described in Non-patent Document 1 or by using the software "TrueAllele" from Cybergenetics, Inc., or the software "GeneMapper" from ABI Cybergenetics, to have possibly been erroneously determined in terms of genotype may be eliminated or shown in a separate color or with a separate type of line.
Function 11: A function based on Function 1 for enlarging an area designated by the user. Specifically, as shown in Fig. 34 indicated by 3400, not just the entire peak lump but also a region of interest, as indicated by 3401 (which, in the example of Fig. 34, is a region between the -2 base to the 2 base relative positions) is enlarged when displayed. Such display allows the user to more easily determine if the waveforms are similar.
Function 12: A function based on Function 1 for allocating a color or a type of line to each fragment length and displaying each peak in the color or with the type of line allocated thereto. A case is now considered where waveforms 3500 to 3503 shown in Fig. 35 are to be displayed in an overlapped manner. It is also assumed that the peak of a size marker is bleeding out at the same location (which, in the example of Fig. 35, is at the 160 base position). When such waveforms are displayed in an overlapped manner in colors allocated in accordance with the fragment length of each peak, a waveform 3504 is obtained. The noise markers caused by the bleeding of the peak of a size marker, as indicated by 3505 to 3507, are all displayed in the same color, although the relative fragment lengths from the true peak are different. Thus, the user can easily see that the noise markers are appearing at the same location among the plurality of waveforms.

The invention provides a gene information display apparatus for displaying the result of analysis of the length of a DNA fragment based on a detection signal from a PCR amplification product thereof according to claim 1. Preferred embodiments are defined in the dependent claims.

In the gene information display apparatus of a preferred embodiment, the result of analysis includes information indicating one or two true peaks in the detection signal from the PCR amplification product that are judged to be indicative of the length of the DNA fragment, wherein the processing unit displays a detection signal from a PCR amplification product that contains two true peaks while performing a correction to equalize the signal intensity of the two true peaks.

In the gene information display apparatus of a preferred embodiment, the result of analysis includes information indicating one or two true peaks that are judged to be indicative of the length of the DNA fragment in the detection signal from the PCR amplification product, wherein the processing unit displays, in a graph in an overlapped manner, the detection signals from the PCR amplification products of only those individuals of which the length of a DNA fragment indicated by the true peak is included in a preset range of fragment lengths.

In the gene information display apparatus of a preferred embodiment, the result of analysis includes information identifying a +A peak, which corresponds to the detection signal of a PCR amplification product to which one adenine is added at the end thereof, wherein the processing unit performs a correction to eliminate the +A peak from the detection signal of the PCR amplification product of each individual, and then displays the detection signal.

In the gene information display apparatus of a preferred embodiment, the result of analysis includes information identifying a +A peak, which corresponds to the detection signal of a PCR amplification product to which one adenine is added at the end thereof, wherein the processing unit displays the +A peak in the detection signal of a PCR amplification product of each individual in a different manner.

In the gene information display apparatus of a preferred embodiment, the processing unit enlarges or reduces in size the display along the horizontal axis such that the fragment length interval of the PCR amplification product of each individual assumes an integer value.

In the gene information display apparatus of a preferred embodiment, the processing unit explicitly displays the height of the detection signal of the PCR amplification product of each individual.

In the gene information display apparatus of a preferred embodiment, the processing unit displays the detection signals from the PCR amplification products of a plurality of individuals in an overlapped manner except for a specific individual.

In the gene information display apparatus of a preferred embodiment, the processing unit displays a specific fragment length range in an enlarged manner.

In the gene information display apparatus of a preferred embodiment, the processing unit displays each detection signal in accordance with a mode allocated to each fragment length.

Also disclosed herein is an apparatus for displaying the result of analysis of the length of a DNA fragment based on a detection signal obtained from a PCR amplification product thereof, comprising:
a processing unit for displaying in a graph a detection signal from the PCR amplification product of an individual and a detection signal expected of the individual in an overlapped manner, wherein the intensity of the detection signal is shown on a first axis and the fragment length is shown on a second axis.

The invention also provides a method for displaying the result of analysis of the length of a DNA fragment based on a detection signal from a PCR amplification product according to claim 12. Preferred embodiments are defined in claims 13 to 22.

In the method for displaying gene information of a preferred embodiment, the result of analysis includes information indicating one or two true peaks in the detection signal from the PCR amplification product that are judged to be indicative of the length of the DNA fragment thereof, the method comprising the step of:
a processing unit displaying a detection signal from a PCR amplification product that contains two true peaks while performing a correction to equalize the signal intensities of the two true peaks.

In the method for displaying gene information of a preferred embodiment, the result of analysis includes information indicating one or two true peaks in the detection signal from the PCR amplification product that are judged to be indicative of the length of the DNA fragment thereof, the method comprising the step of:
the processing unit displaying, in a graph in an overlapped manner, the detection signals from the PCR amplification products of only those individuals of which the length of a DNA fragment indicated by the true peak is included in a preset range of fragment length.

In the method for displaying gene information of a preferred embodiment, the result of analysis includes information identifying a +A peak, which corresponds to the detection signal of a PCR amplification product to which one adenine is added at the end thereof, wherein the processing unit performs a correction to eliminate the +A peak from the detection signal from the PCR amplification product of each individual.

In the method for displaying gene information of a preferred embodiment, the result of analysis includes information identifying a +A peak, which corresponds to the detection signal of a PCR amplification product to which one adenine is added at the end thereof, wherein the processing unit displays the +A peak in the detection signal from the PCR amplification product of each individual in a different manner.

In the method for displaying gene information of a preferred embodiment, the processing unit enlarges or reduces in size the display in the horizontal direction such that the fragment length interval in the PCR amplification product of each individual assumes an integer value.

In the method for displaying gene information of a preferred embodiment, the processing unit explicitly displays the height of the detection signal from the PCR amplification product of each individual.

In the method for displaying gene information of a preferred embodiment, the processing unit displays the detection signals from the PCR amplification products of a plurality of individuals except for a specific individual in an overlapped manner.

In the method for displaying gene information of a preferred embodiment, the processing unit enlarges only a specific fragment length range.

In the method for displaying gene information of a preferred embodiment, the processing unit displays each detection signal in accordance with a mode allocated to each fragment length.

Also disclosed herein is a method for displaying the result of analysis of the length of a DNA fragment based on a detection signal from a PCR amplification product thereof in a computer system equipped with a processing unit and a display unit, the method comprising the step of:
displaying in a graph a detection signal from the PCR amplification product of an individual and a detection signal expected of the individual in an overlapped manner, wherein the intensity of the detection signal is shown on a first axis and the fragment length is shown on a second axis.

The invention also provides a computer program for carrying out the above-described methods for displaying gene information in a computer system equipped with a processing unit and a display unit.

Thus, in accordance with the method and apparatus for displaying gene information according to the invention, a DNA fragment is subjected to PCR amplification and electrophoresis, and waveform data obtained by fluorescence analysis is displayed in such a manner that an experimental error can be easily found by visual inspection.

### BRIEF DESCRIPTION OF THE DRAWINGS

Fig. 1 schematically shows a process of standardizing the vertical and horizontal axes of a waveform graph and then overlapping the waveforms of a plurality of individuals.
Fig. 2 schematically shows a process of overlapping the waveforms from a plurality of individuals in groups in accordance with the relative relationship between alleles.
Fig. 3 schematically shows a process of overlapping the waveforms from a plurality of individuals after performing a correction for equalizing the heights of the two true peaks in a heterozygote.
Fig. 4 schematically shows a process of overlapping the waveforms of only those individuals that are included in a preset range of fragment length.
Fig. 5 schematically shows a process of overlapping the waveforms from a plurality of individuals while eliminating a +A peak.
Fig. 6 shows a functional block diagram of the internal structure of a gene information display system according to an embodiment of the invention.
Fig. 7 shows an example of the structure of experiment data contained in a data memory in the gene information display system of Fig. 6.
Fig. 8 schematically shows a flowchart of processes performed by the gene information display system shown in Fig. 6.
Fig. 9 shows a flowchart illustrating the details of the process for displaying the waveform data of a plurality of individuals in Fig. 8 in an overlapping manner.
Fig. 10 shows a detailed flowchart illustrating the details of the process for extracting a peak set that satisfies a condition designated by the user in Fig. 9.
Fig. 11 shows a flowchart illustrating the details of the process for extracting only those individuals that have alleles in a range of bases designated by the user and counting the number of such individuals shown in Fig. 9
Fig. 12 shows a flowchart illustrating the details of the process for standardizing the waveforms in Fig. 9.
Fig. 13 shows a flowchart illustrating the details of the process for adjusting the height of the peaks in Fig. 9.
Fig. 14 shows a flowchart illustrating the details of the process for displaying only the original peaks in Fig. 9.
Fig. 15 shows a flowchart illustrating the details of the process for displaying a +A peak in separate color in Fig. 9.
Fig. 16 shows a display screen example in the gene information display system shown in Fig. 6.
Fig. 17 shows a display screen example in the gene information display system shown in Fig. 6.
Fig. 18 shows a display screen example in the gene information display system shown in Fig. 6.
Fig. 19 shows a display screen example in the gene information display system shown in Fig. 6.

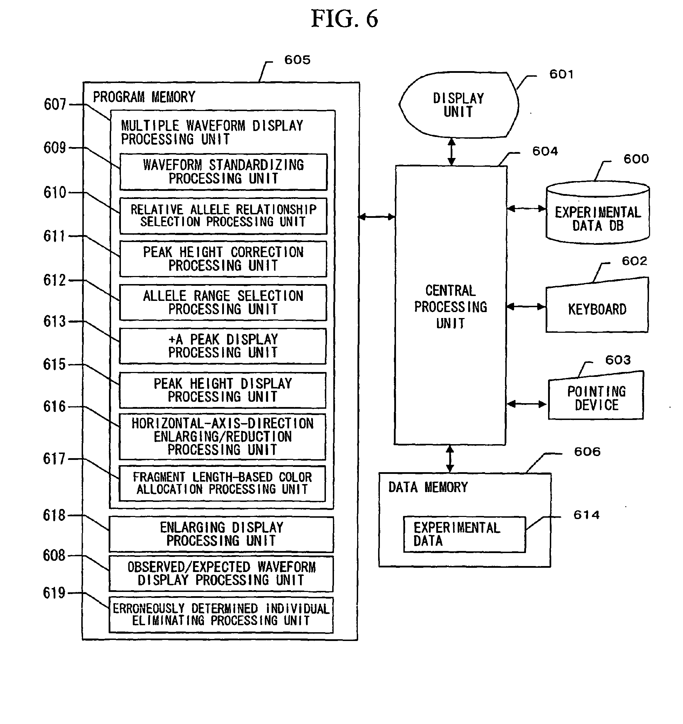

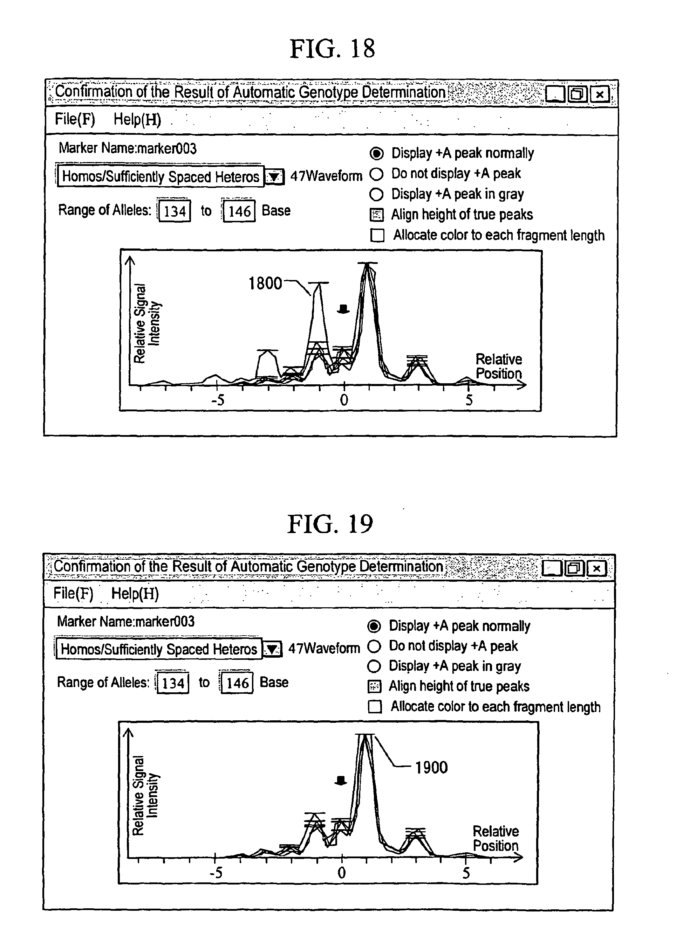

Fig. 19 shows a display screen example in the gene information display system shown in Fig. 6.

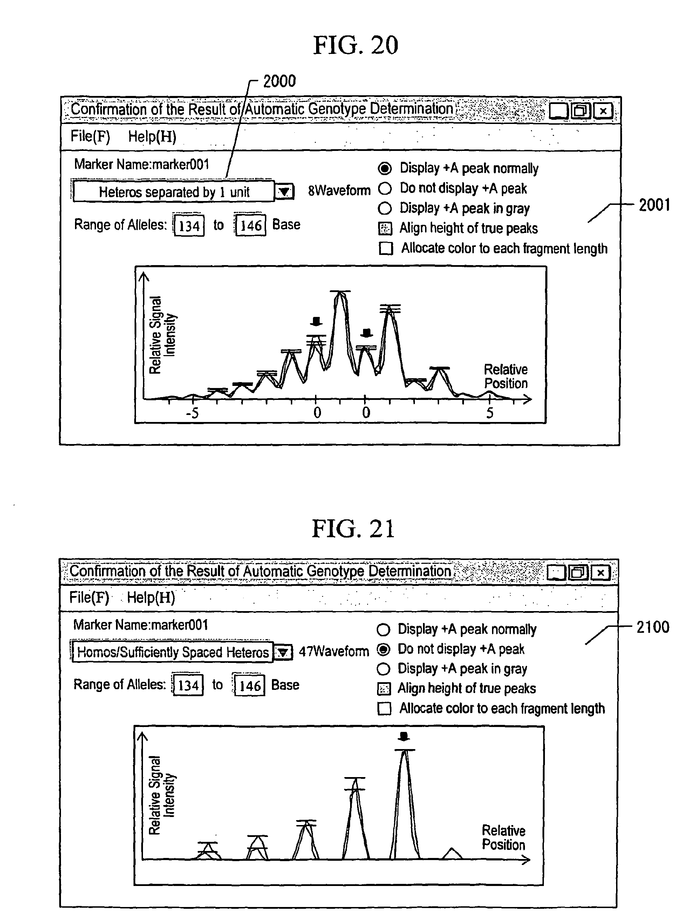

Fig. 20 shows a display screen example in the gene information display system shown in Fig. 6.
Fig. 21 shows a display screen example in the gene information display system shown in Fig. 6.

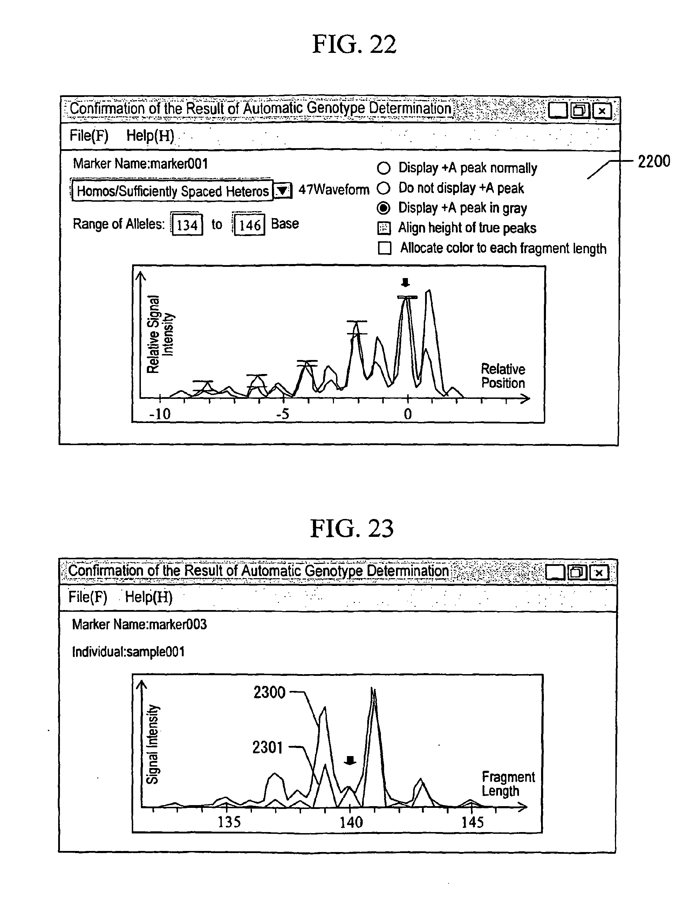

Fig. 22 shows a display screen example in the gene information display system shown in Fig. 6.
Fig. 23 shows a display screen example in the gene information display system shown in Fig. 6.

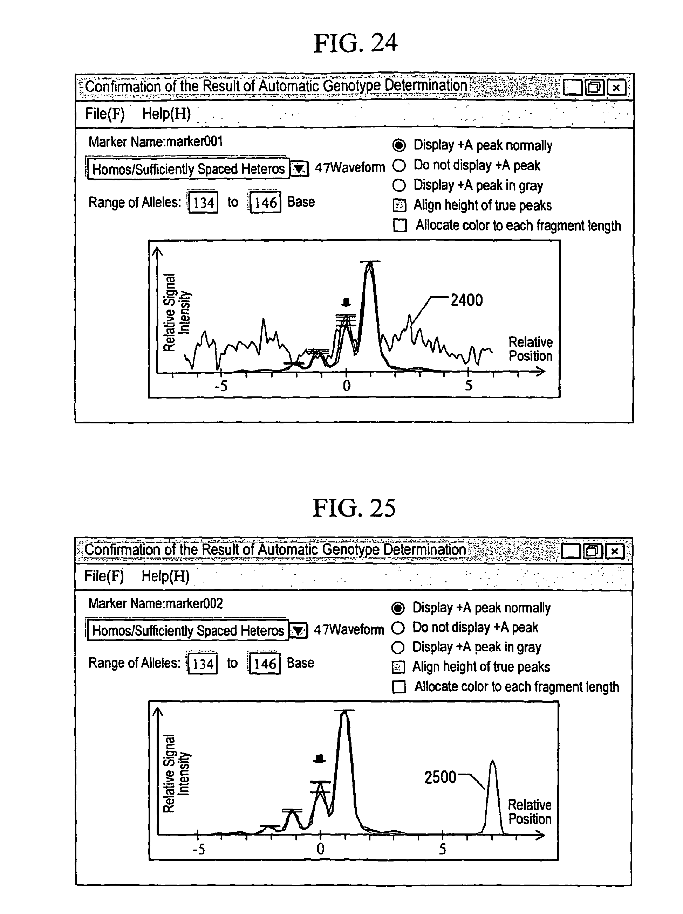

Fig. 24 shows a display screen example in the gene information display system shown in Fig. 6.
Fig. 25 shows a display screen example in the gene information display system shown in Fig. 6.
Fig. 26 shows a drawing for the description of microsatellites that appear on the genome.
Fig. 27 schematically shows an experiment procedure for extracting amplifying a DNA fragment from a microsatellite portion by PCR and electrophoresis.
Fig. 28 shows drawings for the description of a stutter peak and a +A peak, which are typical noise caused in the experiment processes involving PCR and electrophoresis.
Fig. 29 shows waveforms that appear in graphs of the result of fluorescence analysis of homozygotes and heterozygotes.
Fig. 30 shows waveform graphs obtained through experiments involving various errors.
Fig. 31 schematically shows a process of aligning the interval of two true peaks in a heterozygote by enlarging or reducing in size the waveform along the horizontal axis.
Fig. 32 schematically shows a process of adding a display indicating the height of a peak.
Fig. 33 schematically shows a process of overlapping waveforms except for those of an individual designated by the user.
Fig. 34 schematically shows a process of enlarging a range designated by the user.
Fig. 35 schematically shows a process of allocating a color to each fragment length.

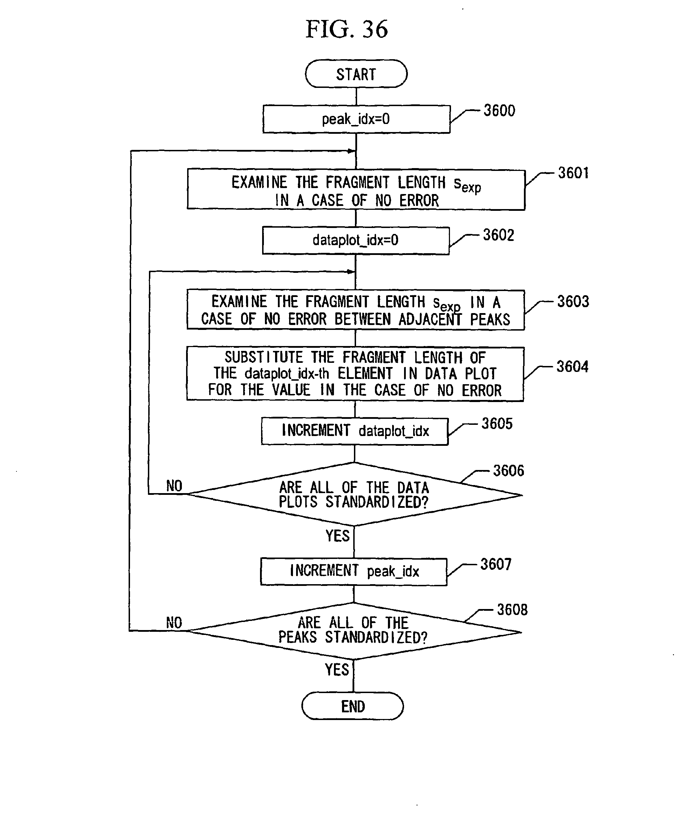

Fig. 36 shows a flowchart illustrating the details of the process of enlarging or reducing in size the waveforms along the horizontal axis in Fig. 9.

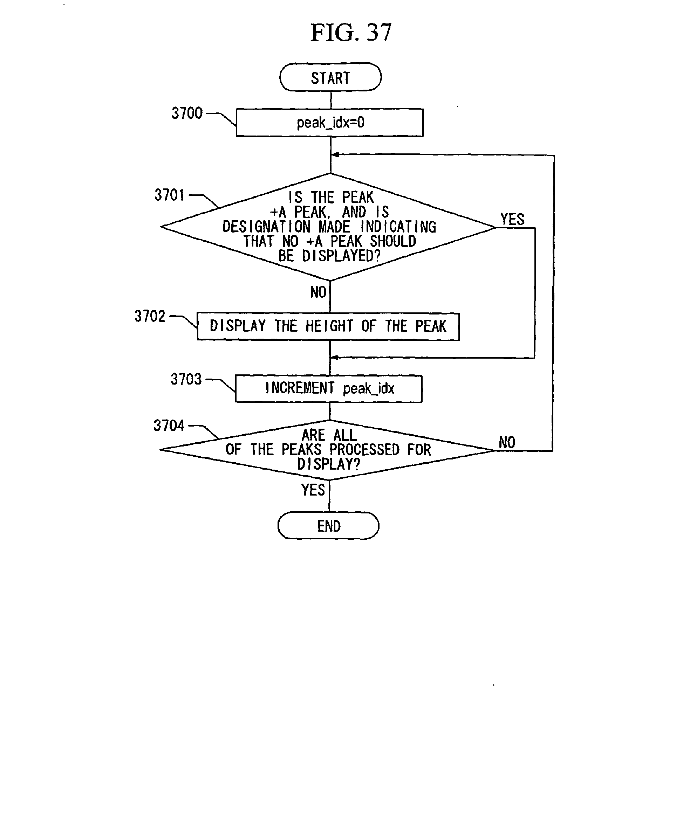

Fig. 37 shows a flowchart illustrating the details of the process of indicating the height of a peak in Fig. 9.

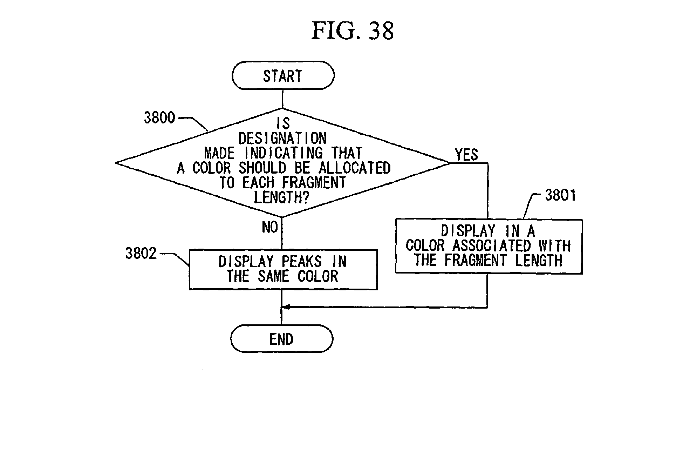

Fig. 38 shows a flowchart illustrating the details of the process of allocating a color to each fragment length in Figs. 9, 14, and 15.

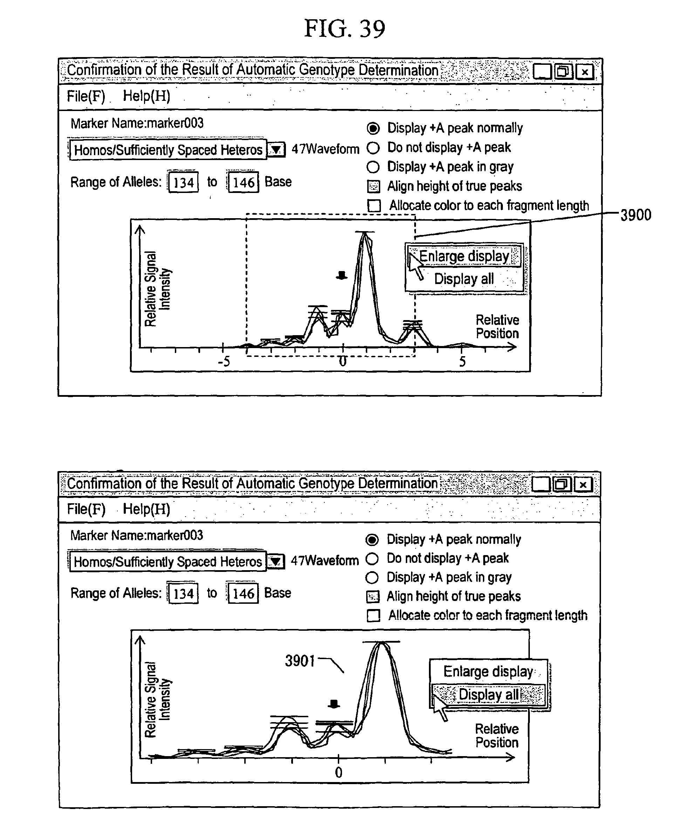

Fig. 39 shows a display screen example in the gene information display system shown in Fig. 6.

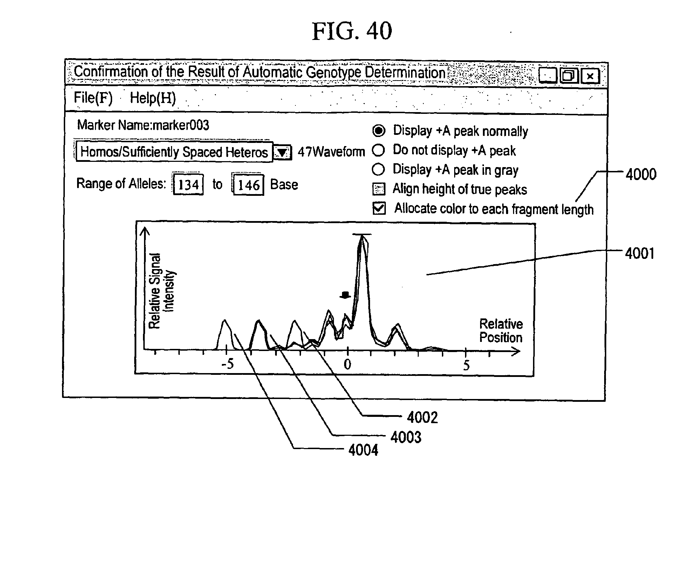

Fig. 40 shows a display screen example in the gene information display system shown in Fig. 6.

### DESCRIPTION OF THE PREFERRED EMBODIMENTS OF THE INVENTION

In the following, a gene information display system will be described with reference to the drawings as a preferred embodiment of the method and apparatus for displaying gene information according to the invention. Figs. 6 to 25 and 36 to 40 show examples of the structure and operation of the gene information display system. Throughout these drawings, like reference numerals identify identical elements with basically identical structure and operation.

### Structure of the Gene Information Display System

Fig. 6 shows a functional block diagram schematically showing the internal structure of a gene information display system according to an embodiment of the invention. This system, which is a gene data interpretation support system, includes an experiment data DB600 in which experimentally obtained data is stored, a display unit 601 for displaying data, a keyboard 602 and a pointing device 603 such as a mouse for performing operations such as selecting a menu in response to displayed data, a central processing unit 604 for performing necessary operating or controlling processes, a program memory 605 for storing programs required by the processes performed by the central processing unit 604, and a data memory 606 in which data required by the processes performed by the central processing unit 604 is stored.

The program memory 605 includes: a multiple waveform display processing unit 607 for performing the aforementioned Function 1; an enlarged display processing unit 618 for performing the aforementioned Function 11; an observed/expected waveform display processing unit 608 for performing the aforementioned Function 7; and an erroneously determined individual removal processing unit 619 for performing the aforementioned Function 10. The multiple waveform display processing unit 607 includes a waveform normalization processing unit 609 for performing Function 2, a relative allele relationship selection processing unit 610 for performing Function 3, a peak height correction processing unit 611 for performing Function 4, an allele range selection processing unit 612 for performing Function 5, a +A peak display processing unit 613 for performing Function 6, a peak height display processing unit 615 for performing Function 9, a horizontal-axis-direction enlargement/reduction processing unit 616 for performing Function 8, and a fragment-length-based color allocation processing unit 617 for performing Function 12. The data memory 606 contains data 614 obtained through an experiment.

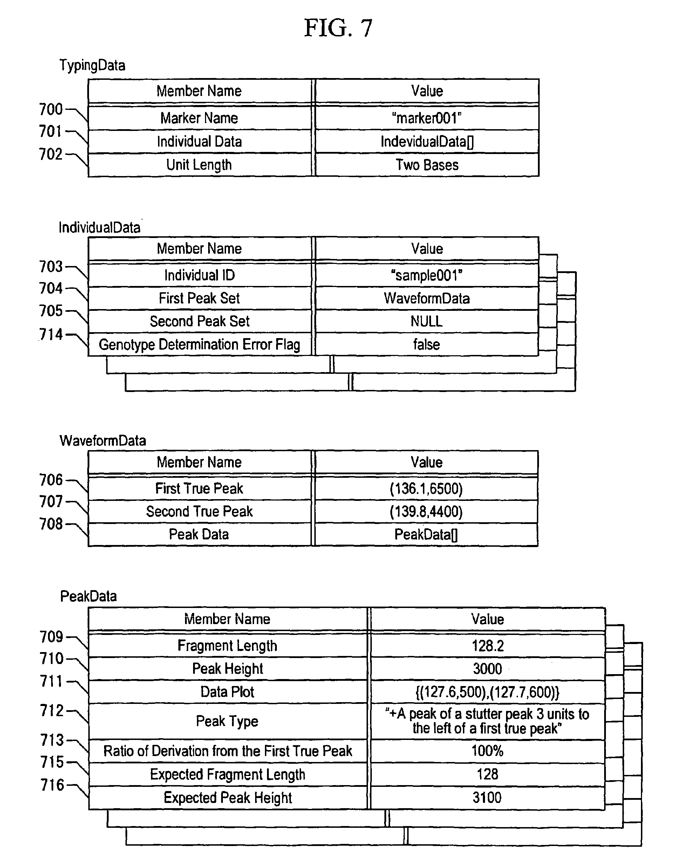

Fig. 7 shows the data structure of the experiment data 614 contained in the data memory 606. This data structure, TypingData, includes a marker name 700, individual data 701, and the unit length 702 of the marker. The individual data 701 is stored in the form of an array according to a data structure, IndividualData, as described below.

The data structure IndividualData includes, for a number i of individuals, individual ID 703, a first peak set 704, a second peak set 705, and a genotype determination error flag 714. In the first and second peak sets 704 and 705, the data is stored in the form of a data structure WaveformData, as described below. The second peak set 705 stores data only for those heterozygotes of which the two true peaks are sufficiently separated, and for the others (homozygotes and those heterozygotes of which the two true peaks are close to each other), it holds a NULL value (i.e., only one peak set is retained). In the genotype determination error flag 714, all individuals are initialized with "false."

The data structure WaveformData includes a first true peak 706, a second true peak 707, and peak data 708. The peaks 706 and 707 each carry a pair of values of fragment length and peak height. The peak data 708 carries data in the form of an array according to a data structure PeakData, as described below. The second true peak 707 carries data only for those heterozygotes of which the two true peaks are close to each other, and for the others, (homozygotes and those heterozygotes of which the two true peaks are sufficiently separated), it carries a NULL value (i.e., there is only one true peak contained in the peak set).

The data structure PeakData includes, for a number j of peaks, fragment length 709, peak height 710, data plot 711, peak type 712, the ratio of derivation from the first true peak 713, fragment length 715 in an expected waveform, and peak height 716 in the expected waveform. The data plot 711 consists of a set of values of the X coordinate (fragment length) and the associated Y coordinate (signal intensity). In the data plot 711 of Fig. 7, the signal intensity at 127.6 base is 500, and the signal intensity at 127.7 base is 600, for example. The peak type 712 indicates either a true peak, a stutter peak, or a +A peak for each peak. In the case of a stutter peak, it shows the distance from a true peak in units. In the case of a +A peak, it indicates whether it derives from a true peak or a stutter peak. And in the case where two true peaks are included in the peak set, the data indicates which of the true peaks the peak derives from. In the case where peaks deriving from two true peaks are overlapped, the two are concatenated with each other when they are stored. When it is determined that the peak does not derive from either of the true peaks, as shown by the peak at the far right of (4) in Fig. 30, a value "Other" is carried. When the data herein indicates that the peak is a first true peak or a second true peak, the value of the peak height 710 is equal to the peak height of either the first true peak 706 or that of the second true peak 707 in the WaveformData. The identity of each peak can be estimated by referring to Non-patent Document 2, as mentioned with reference to the Background Art. Fig. 7 shows an example of a +A peak of a stutter peak (that appears on the left in the waveform) in which the number of repetition has decreased by 3 units from the first true peak. Numeral 713 indicates whether and how much each peak derives from the first or the second true peak, which, in Fig. 7, shows the value of 100% since the particular peak derives solely from the first true peak. The same is true in the case where there is only one true peak. Conversely, when the peak derives solely from the second true peak, the value would be 0%. When components from two true peaks are composed, the value would be an intermediate value. The calculation of the derivation ratio can be made by referring to Non-patent Document 2, as mentioned with reference to the Background Art. Numerals 715 and 716 indicate the fragment length and peak height, respectively, in a waveform that is expected on the assumption that the first true peak 706 and the second true peak 707 (when not NULL values) in the WaveformData are true peaks. The expected fragment length and peak height can be calculated by referring to Non-patent Document 2, as mentioned with reference to the Background Art.

### Operation of the Gene Information Display System

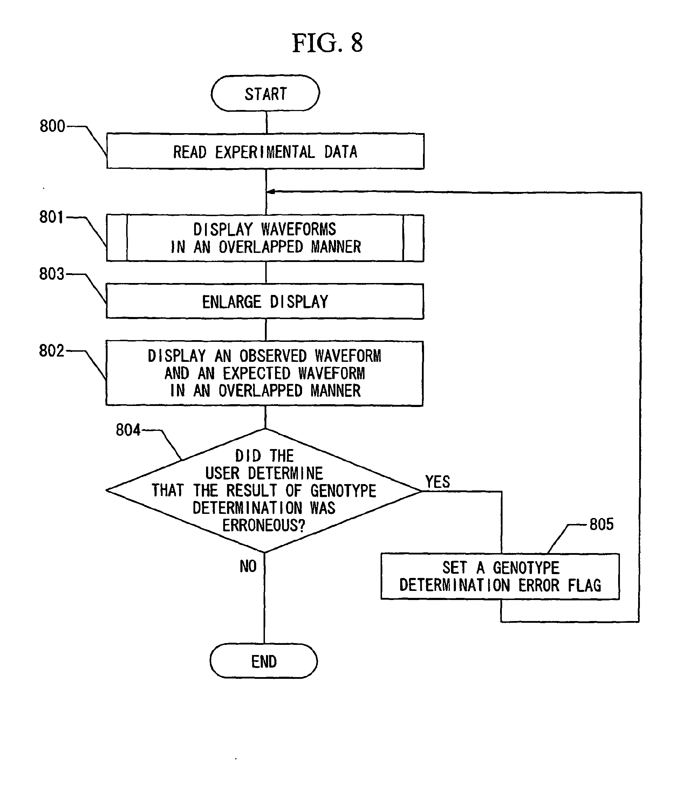

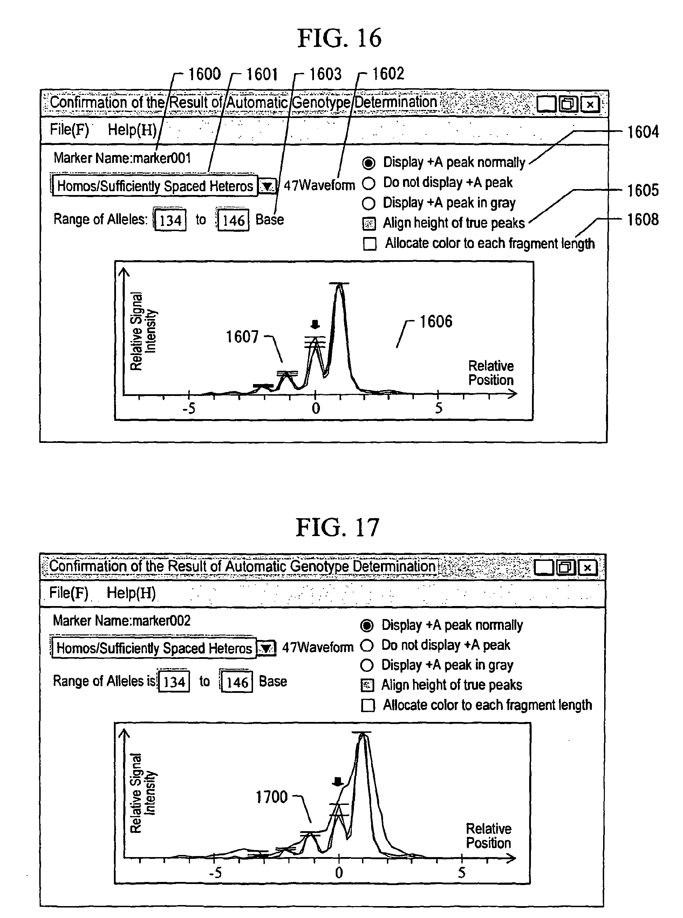

In the following, the operation of the gene information display system that is constructed as described above is described. Fig. 8 shows a flowchart schematically illustrating the flow of processes performed by the gene information display system shown in Fig. 6. In Fig. 8, the gene information display system reads the experiment data from the experiment data DB600 in the form of the data structure TypingData (step 800). The system then carries out a process to overlap waveform data from a plurality of individuals, and displays a screen on which the overlapped waveform data is shown to the user so that he or she can confirm at once whether or not the result of automatic determination is appropriate and whether or not there are individuals that need a re-experiment (step 801). This process is performed by the multiple waveform display processing unit 607 included in the program memory 605. Concrete examples of the display screen will be described in detail later with reference to Figs. 16-22, 24, 25, and 40. Thereafter, an enlarged display is made for an area designated by the user (step 803). This process is performed by the enlarged display processing unit 618 included in the program memory 605. Concrete examples of the display screen will be described in detail later with reference to Fig. 39. Thereafter, a display is made in which the observed waveform and an expected waveform for the individual designated by the user are overlapped (step 802). This process is performed by the observed/expected waveform display processing unit 608 included in the program memory 605. Concrete examples of the display screen will be described in detail later with reference to Fig. 23. Then, it is checked whether or not the user has indicated that the result of genotype determination is erroneous as a result of visual inspection (step 804). If the user has indicated such error, the genotype determination error flag 714 for a relevant individual is set to "true" (step 805). These processes are performed by the erroneously determined individual removal processing unit 619 included in the program memory 605. Thereafter, the routine returns to step 801, where the process is repeated again.

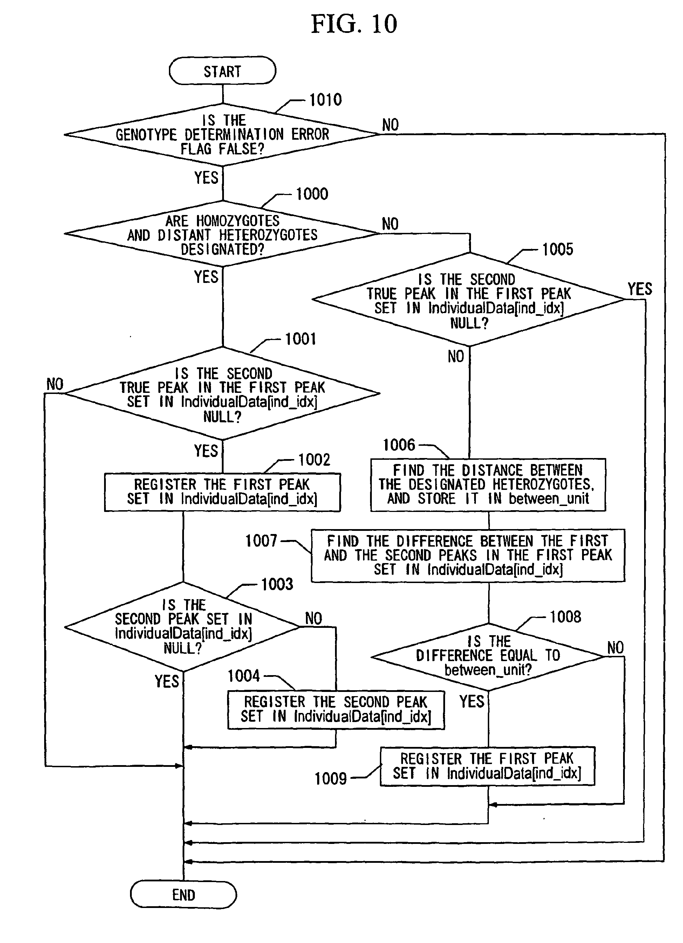

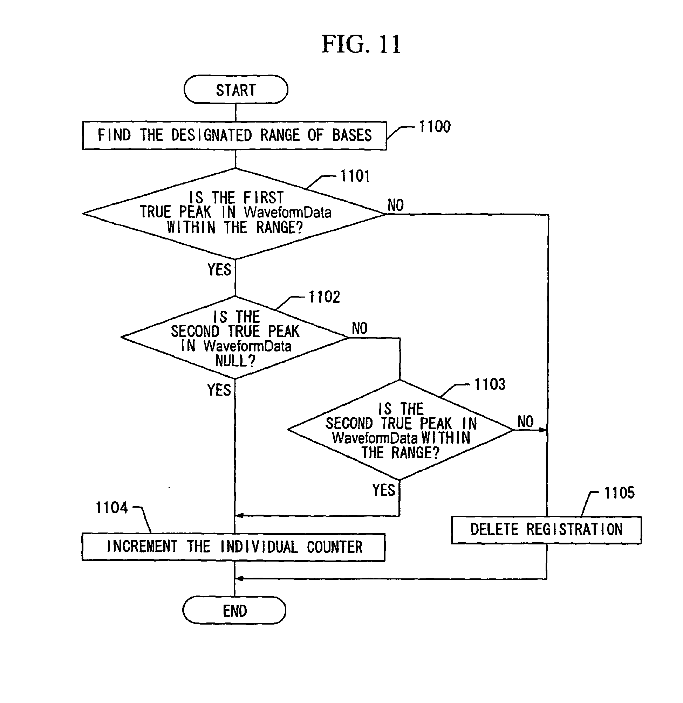

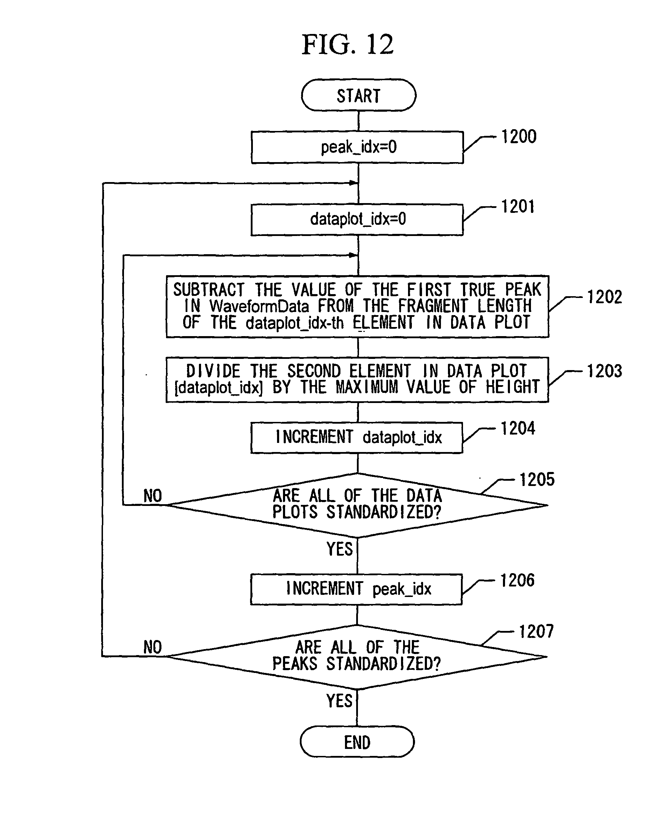

Fig. 9 shows a flowchart illustrating the details of the process for displaying the waveform data from a plurality of individuals in an overlapped manner in step 801 of Fig. 8. In Fig. 9, first, as described with reference to Function 3, a peak set that matches the condition designated by the user is extracted from the peak sets 704 and 705 in the experiment data 614 contained in the data memory 606 (step 900). This process is performed by the allele relationship selection processing unit 610, as will be described later with reference to Fig. 10. Then, as described with reference to Function 5, only those individuals that have alleles within the base range designated by the user are extracted, and the number of such individuals is counted (step 901). This process is performed by the allele range selection processing unit 612, as will be described later with reference to Fig. 11. The number of the peak sets thus extracted is shown on the screen, as will be described later (1602 in Fig. 16). Further, as described with reference to Function 2, waveform standardization is carried out (step 902). This process is performed by the waveform normalization processing unit 609, as will be described later with reference to Fig. 12. Thereafter, as described with reference to Function 8, the waveform is enlarged or reduced in size along the horizontal axis (step 910). This process is carried out by the horizontal-axis-direction enlargement/reduction processing unit 616, as will be described later with reference to Fig. 36.

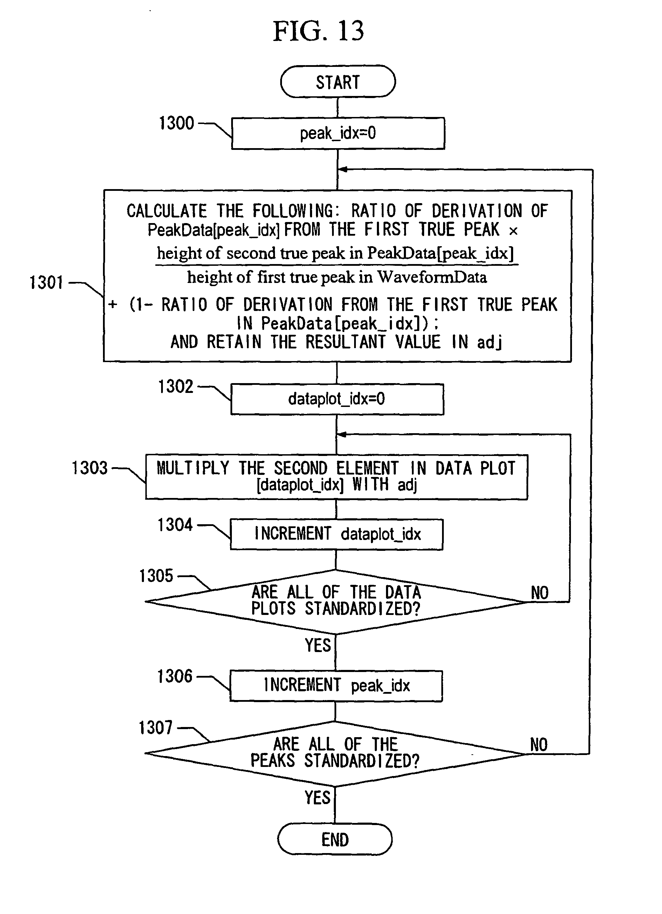

It is then examined whether or not the user has designated heterozygotes of which the two true peaks are close to each other and also made a designation indicating that the height of the true peaks should be made uniform when displayed (step 903). If such designations are made (i.e., if a check box 2001 shown in Fig. 20 is selected, as will be described later), the height of the peaks is adjusted, as described above with reference to Function 4 (step 904). This process is performed by the peak height correction processing unit 611, as will be described later with reference to Fig. 13.

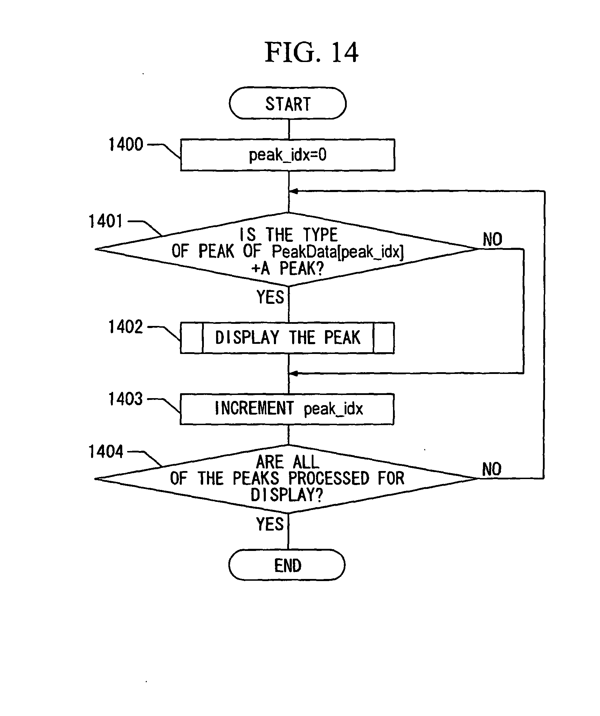

Then, it is examined whether or not the user has made a designation indicating that a +A peak should not be displayed (step 905). If such designation has been made (i.e., if a radio button 2100 shown in Fig. 21, which will be described later, is selected instructing that the +A peak be not displayed), only the original peaks (a true peak and a stutter peak) are displayed (step 906) as described above with reference to Function 6. This process is performed by the +A peak display processing unit 613, the detail of which will be described later with reference to Fig. 14.

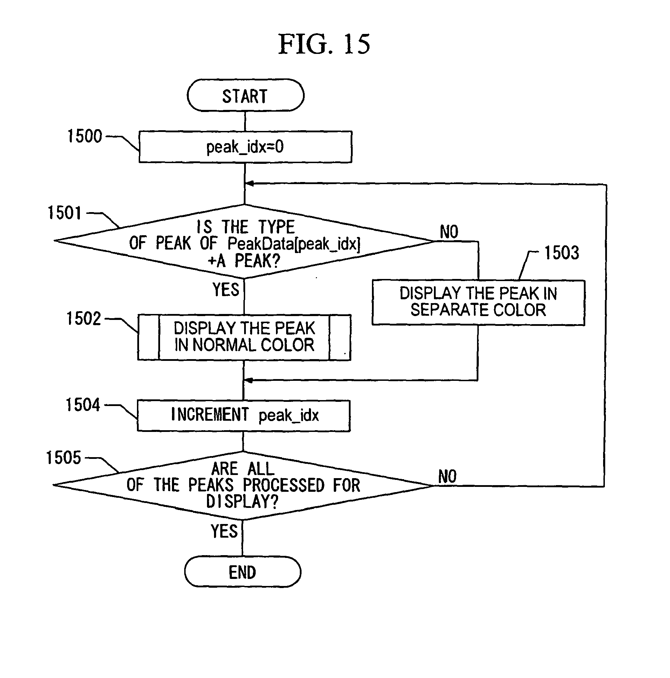

If in step 905 the user has not made the designation indicating that the +A peak should not be displayed, it is further examined whether or not the user has made a designation indicating that the +A peak should be displayed in a separate color (step 907). If such a designation is made (i.e., if a radio button 2200 shown in Fig. 22, which will be described later, is selected to indicate that the +A peak should be displayed in a separate color), the +A peak is displayed in a separate color (step 908), as described above with reference to Function 6. This process is performed by the +A peak display processing unit 613, the detail of which will be described later with reference to Fig. 15.

If in step 907 the user has not made the designation indicating that the +A peak should be displayed in a separate color, all of the peaks are displayed (step 909). This process is performed by the fragment-length-based color allocation processing unit 617, the detail of which will be described later with reference to Fig. 38. In this step, when the waveforms of heterozygotes that are close to each other are displayed, the two true peaks are adjusted to be positioned at zero on the X axes, as shown in Figs. 36 and 20, which will be described later.

Thereafter, as described with reference to Function 9, a display is made in which the height of the peaks is shown (step 911). This process is performed by the peak height display processing unit 615, the details of which will be described later with reference to Fig. 37.

Fig. 10 shows a flowchart illustrating the details of the process of extracting a peak set that matches a condition designated by the user in step 900 shown in Fig. 9. While this extraction process is carried out for each individual, the following example concerns the extraction from an ind_idx-th individual. In Fig. 10, it is first examined whether or not the genotype determination error flag 714 is false (step 1010). If it is set to false, it is then examined whether or not a designation is made indicating "only homozygotes and those heterozygotes in which the two alleles are sufficiently separated" (step 1000). If there is such a designation (i.e., if such a designation is made in a pull-down menu 1601 shown in Fig. 16, as will be described later), it is examined whether or not the second true peak 707 in the first peak set 704 in IndividualData[ind_idx] is NULL (step 1001). If it is NULL, the peak set has just one true peak, i.e., it corresponds to a homozygote or a heterozygote in which the two alleles are sufficiently separated. In this case, the first peak set 704 in the IndividualData[ind_idx] is registered as a subject of extraction (step 1002). Thereafter, it is examined whether or not the second peak set 705 in the IndividualData[ind_idx] is NULL (step 1003). If it is not NULL, the peak set corresponds to a heterozygote in which the two alleles are sufficiently separated. Therefore, the second peak set 705 in the IndividualData[ind_idx] is registered as a subject of extraction (step 1004).

On the other hand, in step 1000, if heterozygotes in which the two alleles are close to each other are designated, it is examined whether or not the second true peak 707 in the first peak set 704 in the IndividualData[ind_idx] is NULL (step 1005). If it is not NULL, the peak set has two true peaks, i.e., it corresponds to a heterozygote in which the true peaks are close to each other. In this case, it is examined what the designated distance of heterozygotes is in terms of units (by examining the value in the pull-down menu 1601 in Fig. 16), and the result is retained as a variable "between_unit" (step 1006). Then, the difference between the fragment length of the first true peak 706 and that of the second true peak 707 in the first peak set 704 in the IndividualData[ind_idx] (step 1007) is determined. This is followed by examining whether or not the difference is equal to the product of the "between_unit" and the unit length (step 1008). If it is, the first peak set 704 in the IndividualData[ind_idx] is registered as a subject of extraction (step 1009).

Fig. 11 shows a flowchart illustrating the details of the process for extracting only those individuals that have alleles in a range of bases designated by the user in step 901 of Fig. 9 and counting the number of such individuals. While this process is carried out for each of the peak sets extracted in step 900, the following example concerns the processing of a single peak set. In Fig. 11, first the range of bases designated by the user (in the text box 1603 in Fig. 16) is examined (step 1100). Then, it is examined whether or not the fragment length of the first true peak 706 in the WaveformData is within the range determined in step 1100 (step 1101). If it is within the range, it is then examined whether the second true peak 707 in the WaveformData is NULL (step 1102). If it is not NULL, it is further examined whether or not the fragment length of the second true peak 707 in the WaveformData is within the range determined in step 1100 (step 1103). If it is, or if the examination in step 1102 indicates NULL, the relevant individual is considered to be appropriate as a subject of extraction, and an individual counter for counting the number of extracted individuals is incremented by one (step 1104). If in step 1101 it is determined that the fragment length of the first true peak 706 is not within the designated range, or if in step 1103 it is determined that the fragment length of the second true peak 707 is not within the designated range, the relevant individual is considered to be not included in the subjects for extraction, and its registration is deleted (step 1105).

Fig. 12 shows a flowchart illustrating the details of the process for standardizing the waveform in step 902 in Fig 9. While this waveform standardizing process is carried out for each of the peak sets extracted in steps 900 and 901, the following description concerns an example in which the process is performed on a single peak set. In Fig. 12, the index variable peak_idx, which indicates the peak number, is initialized with zero (step 1200). Then, the index variable dataplot_idx, which indicates the data plot number in the peak, is initialized with zero (step 1201). Thereafter, the fragment length of the first true peak 706 in WaveformData is subtracted from the fragment length of the dataplot_idx-th element in the data plot 711 of PeakData[peak_idx] 708 in WaveformData (step 1202). The peak height of the dataplot_idx-th element in the data plot 711 of PeakData[peak_idx] 708 in WaveformData is divided by the maximum value of the height in this peak set (step 1203). The maximum height value can be obtained by examining the maximum values of the peak heights of all of the data plots in all peaks. Thereafter, the variable dataplot_idx is incremented by just 1 (step 1204). It is then determined whether or not all of the data plots have been standardized, i.e., whether or not the dataplot_idx is equal to or more than the number of data plots (step 1205). If there are data plots yet to be standardized, the routine returns to step 1202 where the process is repeated once again. If all of the data plots have been standardized, the variable peak_idx is incremented by just one (step 1206). It is then determined whether or not all of the peaks have been standardized, i.e., whether or not the variable peak_idx is equal to or more than the number of peaks (step 1207). If there are peaks left that have yet to be standardized, the routine returns to step 1201 where the process is repeated once again.

Fig. 36 shows a flowchart illustrating the details of the process for enlarging or reducing the waveform in size along the horizontal axis in step 910 of Fig. 9. While this horizontal enlarging/reducing process is carried out for each of the peak sets extracted in steps 900 and 901, the following example concerns the processing of a single peak set. In Fig. 36, first the index variable peak_idx, which shows the peak number, is initialized with zero (step 3600). Then, concerning the fragment length of a peak_idx-th peak, a value sₑₓₚ that is expected in the absence of error is checked (step 3601). This process corresponds to the examination of the expected fragment length 715 in the PeakData[peak_idx] 708 in WaveformData. Thereafter, the index variable dataplot_idx, which shows the data plot number in the peak, is initialized with zero (step 3602). This is followed by the examination of a value sₑₓₚ' for the fragment length of an adjacent peak that is expected in the absence of error (step 3603). This process corresponds to the examination of an expected fragment length 715 for the peak_idx-1-th peak that is adjacent to the left if the dataplot_idx-th element is to the left of the top of the peak, or for the peak_idx+1-th peak that is adjacent to the right of the peak if the element is to the right of the top of the peak. Thereafter, the fragment length of the dataplot_idx-th element in the data plot is set to the value expected in the absence of error (step 3604). This calculation can be made by determining an internally dividing point assuming that the data plots are located at regular intervals between sₑₓₚ and S_{exp'}. The value is then regarded as the fragment length (the first element) of the dataplot_idx-th element in the data plot 711. Thereafter, the variable dataplot_idx is incremented by just one (step 3605). It is then determined whether or not all of the data plots have been standardized, i.e., whether or not the dataplot_idx is equal to or more than the number of data plots (step 3606). If there are data plots left that are yet to be standardized, the routine returns to step 3603 where the process is repeated once again. If the fragment length has been calculated for all of the data plots, the variable peak_idx is incremented by just one (step 3607). This is followed by the determination of whether or not the horizontal enlargement/reduction process has been carried out for all of the peaks, i.e., whether or not the variable peak_idx is now equal to or more than the number of peaks (step 3608). If there are peaks that are yet to be subjected to the horizontal enlarging/reducing process, the routine returns to step 3601 where the process is repeated once again.

Fig. 13 shows a flowchart illustrating the details of the process for adjusting the height of the peak in step 904 of Fig. 9. While this peak height adjusting process is carried out for each of the peak sets extracted in steps 900 and 901, the following example concerns the processing of a single peak set. In Fig. 13, first the index variable peak_idx, which shows the peak number, is initialized with zero (step 1300). Then, the following calculations are made: (the ratio of derivation from the first true peak 713 in the PeakData[peak_idx] 708 in WaveformData from) * {(the peak height of the second true peak 707 in WaveformData) / (the peak height of the first true peak 706 in WaveformData)}+{1-(the ratio of derivation from the first true peak 713 in the PeakData[peak_idx] 708 in WaveformData)}. The result of the calculations is retained as a variable adj (step 1301). Thereafter, the index variable dataplot_idx, which shows the data plot number in the peak, is initialized with zero (step 1302). The peak height of the dataplot_idx-th element of the data plot 711 of PeakData[peak_idx] 708 in WaveformData is then multiplied with the value of variable adj (step 1303). After the variable dataplot_idx is incremented by just 1 (step 1304), it is determined whether or not the height adjustment has been performed for all of the data plots, i.e., whether or not the dataplot_idx is equal to or more than the number of data plots (step 1305). If there are data plots left that are yet to be adjusted for height, the routine returns to step 1303 where the process is repeated once again. If all of the data plots have been subjected to the height adjustment process, the variable peak_idx is incremented by just one (step 1306). It is then determined whether or not all of the peaks have been subjected to the height adjustment process, i.e., whether or not the variable peak_idx is now equal to or more than the number of peaks (step 1307). If there are peaks yet to be adjusted for height, the routine returns to step 1301 where the process is repeated once again.

Fig. 14 shows a flowchart illustrating the details of the process for displaying only the original peak (true peak and stutter peak) in step 906 of Fig. 9. While this process is carried out for each of the peak sets extracted in steps 900 and 901, the following example concerns the processing of a single peak set. In Fig. 14, first the index variable peak_idx, which shows the peak number, is initialized with zero (step 1400). Then, it is determined whether or not the peak type 712 of the PeakData[peak_idx] 708 in WaveformData is a +A peak (step 1401). If it is not a +A peak, the peak is displayed (step 1402). This process is carried out for each fragment length by the fragment-length-based color allocation processing unit 617, the details of which will be described later with reference to Fig. 38. If the waveforms of heterozygotes close to each other are displayed, as described with reference to Fig. 36 and as shown in Fig. 20 to be described later, the two true peaks are adjusted to be positioned at zero on the X axis. Thereafter, the variable peak_idx is incremented by just one (step 1403). It is then determined whether or not the display process has been carried out for all of the peaks, i.e., whether or not the variable peak_idx is equal to or more than the number of peaks (step 1404). If there are peaks left that are yet to be subjected to the display process, the routine returns to step 1401 where the process is repeated once again.

Fig. 15 shows a flowchart illustrating the details of the process for displaying the +A peak in a separate color in step 908 of Fig. 9. While this process is carried out for each of the peak sets extracted in steps 900 and 901, the following example concerns a case where the process is carried out for a single peak set. In Fig. 15, first the index variable peak_idx, which shows the peak number, is initialized with zero (step 1500). Then, it is determined whether or not the peak type 712 of the PeakData[peak_idx] 708 in WaveformData is a +A peak (step 1501). If it is not a +A peak, the peak is displayed in normal color (step 1502). This process is carried out by the fragment-length-based color allocation processing unit 617, the details of which will be described later with reference to Fig. 38. If the peak is a +A peak, it is displayed in a separate color (step 1503). When in steps 1502 and 1503 the waveforms of adjacent heterozygotes are displayed, as mentioned with reference to Fig. 36, and as shown in Fig. 20 which will be described later, the two true peaks are adjusted to be positioned at zero on the X axis. Thereafter, the variable peak_idx is incremented by just one (step 1504). It is then determined whether or not all of the peaks have been subjected to the display process, i.e., whether or not the variable peak_idx is now equal to or more than the number of peaks (step 1505). If there are peaks left that are yet to be subjected to the display process, the routine returns to step 1501 where the process is repeated once again.

Fig. 37 shows a flowchart illustrating the details of the process for displaying the height of the peak in step 911 of Fig. 9. While this process is carried out for each of the peak sets extracted in steps 900 and 901, the following description concerns a case where the process is carried out for a single peak set. In Fig. 37, first the index variable peak_idx, which shows the peak number, is initialized with zero (step 3700). Then, it is determined whether or not the peak type 712 of the PeakData[peak_idx] 708 in WaveformData is a +A peak and whether or not the user has made a designation indicating that +A peaks should not be displayed (step 3701). If these conditions are not satisfied (i.e., if the peak type 712 of the PeakData[peak_idx] 708 in WaveformData is not a +A peak, or if the user has made a designation indicating that a +A peak should be normally displayed, or if the user has made a designation indicating that a +A peak should be displayed in a separate color), the height of the peak_idx-th peak is displayed (step 3702). The display of the height of the peak is made at the position of the peak height 710 of the PeakData[peak_idx] 708 in WaveformData. Thereafter, the variable peak_idx is incremented by just one (step 3703). This is followed by the determination of whether or not the display of height has been made for all of the peaks, i.e., whether or not the variable peak_idx is now equal to or more than the number of peaks (step 3704). If there are peaks left for which the display of height has not been made, the routine returns to step 3701 where the process is repeated once again.

Fig. 38 shows a flowchart illustrating the details of the process for displaying the peak in step 909 of Fig. 9, the process for displaying the peak in step 1402 of Fig. 14, and the process for displaying the peak in a normal color in step 1502 of Fig. 15. In Fig. 9, while this process is carried out for all of the peaks, the following description concerns a case where the process is carried out for the peak_idx-th peak. In Fig. 38, first it is determined whether or not there is the designation indicating that a different color should be allocated to each fragment length (step 3800). If there is such designation (i.e., if such a designation is made in the check box 1608 in Fig. 16, which will be described later), the display is made using colors allocated to each fragment length (step 3801). If there is no such designation, all of the peaks are displayed in the same color (step 3802).

### Example of the Display Screen in the Gene Information Display System

An example of the screen displayed in the above-described gene information display system is described. The display allows the user to easily determine whether or not there is an individual having a different waveform from those of the other individuals from among the waveforms of a number of individuals. The user is also allowed to easily examine the tendency of the waveform of a particular marker and determine whether or not it is necessary to carry out an experiment again for each waveform. Thus, the user only needs to focus his or her attention on the individual whose waveform is different from that of the other individuals, confirm whether the result of automatic genotype determination is appropriate, and then examine the tendency of the waveform of that particular marker. In this way, the user is freed from the need to conduct the troublesome work of confirming the appropriateness of the result of automatic determination for each of the individuals and then making a visual inspection to decide whether or not a re-experiment is required. In this way, the operational burden on the user is greatly reduced.

In the display screen example shown in Fig. 16, 1600 designates a marker name for which an experiment has been conducted. Further, as indicated in 1601, the example shows only homozygotes and those heterozygotes among the individuals subjected to the experiment in which the two alleles are sufficiently separated. The conditions that can be selected in 1601 include, in addition to "only homozygotes and those heterozygotes in which the two alleles are sufficiently separated," as shown in Fig. 16 by way of example, "those individuals that are heterozygotes in which the two alleles are separated by X units," where X is an integer of 1 or greater. In accordance with this condition, only those heterozygotes are displayed in which the two alleles are close to each other, indicating that the stutter or +A peaks are thought to overlap. Numeral 1602 designates the number of waveforms that satisfy the condition. Further, as indicated in 1603, the example shows only those individuals whose fragment lengths are within the designated range. Also, as indicated in 1604, both +A peaks and original peaks are displayed in the same manner. Using 1605, it is possible to make a designation indicating whether or not, in a heterozygote, the heights of the two true peaks should be adjusted to be equal when they are displayed. As indicated in 1605, because no such designation is required for homozygotes, no change can be made. As indicated in 1608, a designation can be made indicating that a color should be allocated to each fragment length (the example of Fig. 16 shows the screen where no such designation is made). In the illustrated example, because, as indicated in 1606, similar waveforms are observed for all of the individuals, it can be determined that no visual inspection is necessary to determine, for each individual, whether or not the result of automatic determination is appropriate and whether or not a re-experiment should be conducted. An indication 1607, which explicitly shows the peak height, allows the user to easily determine whether or not the waveforms are similar.

The display screen example shown in Fig. 17 shows a case where the separation of the peaks is not perfect, such that an individual (1700) whose waveform is different from those of the other individuals is included. In this case, it can be judged that it is only necessary to conduct a visual inspection only for the particular individual 1700 to see whether or not the result of automatic determination is appropriate and whether or not a re-experiment is necessary.

The display screen example shown in Fig. 18 shows a case where an individual (1800) whose waveform is different from those of the other individuals due to the high stutter peak is included. In this case, it can be judged that it is necessary to conduct a visual inspection only for the individual 1800 to see whether or not the result of automatic determination is appropriate, and whether or not a re-experiment is required.

The display screen example shown in Fig. 19 shows a case where an individual (1900) whose fluorescence signal was so strong as to exceed the measurement limit of the detector is included. In this case, it can be judged that it is only necessary to conduct a visual inspection only for the individual 1900 to see whether or not the result of automatic determination is appropriate and whether or not a re-experiment is required.

The display screen example shown in Fig. 20 shows a case where only those individuals that are separated by 1 unit are displayed, in accordance with the designation in 2000. As indicated in 2001, a designation can be made as to whether or not the heights of the two true peaks should be adjusted to be equal when they are displayed. In the heterozygous individuals, there exist two fragment lengths that are estimated to be true peaks used as a reference on the horizontal axis. Therefore, in the range to the left of the left true peak, the value of the fragment length standardized by the waveform normalization processing unit 609 and the horizontal-axis-direction enlargement/reduction processing unit 616 is displayed as the scale on the horizontal axis, while in the range between the left and right true peaks, no scale is shown on the horizontal axis. In the range to the right of the right true peak, a value obtained by subtracting the difference between the expected fragment lengths of the two true peaks from the value of the fragment length standardized by the waveform normalization processing unit 609 and the horizontal-axis direction enlargement/reduction processing unit 616 is used as the scale on the horizontal axis.

The display screen example shown in Fig. 21 shows a case where, as indicated in 2100, no +A peak is displayed and instead only an original peak is displayed. In the display screen example shown in Fig. 22, as indicated in 2200, a +A peak is indicated in a separate color.

The display screen example shown in Fig. 23 shows a case where a waveform 2300 of a single individual observed experimentally and an expected waveform 2301 are displayed in an overlapped manner. This allows the user to easily conduct a visual inspection to see if the waveform 2300 is appropriate. The expected waveform 2301 is displayed with reference to an expected fragment length 715 of PeakData and an expected peak height 716.

The display screen example shown in Fig. 24 shows a case where an individual (2400) is included whose fluorescence signal is so weak that it is buried in the background noise. In this case, it can be judged that it is only necessary to conduct a visual inspection for the individual 2400 to see whether or not the result of automatic determination is appropriate and whether or not a re-experiment is required.

The display screen example shown in Fig. 25 shows a case where an individual (2500) is included in which the peak of a size marker is bleeding out. In this case, it can be judged that it is only necessary to conduct a visual inspection for only the individual 2500 to see if the result of automatic determination is appropriate and if a re-experiment is required.

In the display screen example shown in Fig. 39, of the waveforms displayed in an overlapped manner, a region 3900 designated by the user is enlarged in the display. This operation allows the user to visually inspect the individual waveforms in greater detail in an area designated by the user using a pointing device 603 such as a mouse, as indicated by 3901.

The display screen example shown in Fig. 40 shows an example in which the user has given an instruction that, as indicated by 4000, a color should be allocated to each fragment length. In this case, as indicated by 4001, each peak is displayed in the color allocated to each fragment length. The noise peaks indicated by 4002 to 4004 are all displayed in the same color, making it easy to see that the peaks are all appearing at the same fragment length positions.

As described above, the user can easily determine the tendency of the waveform of each marker using the screens shown in Figs. 16 to 22, 24, 25, 39, and 39. Further, the user can also conduct a visual inspection of each individual to see, using the screen of Fig. 23, whether or not the result of automatic determination is appropriate and whether or not a re-experiment should be conducted. For example, the user only needs to conduct a visual inspection on the individuals 1700, 1800, 1900, 2400, 2500, 4002, 4003, and 4004, which have waveforms different from those of the other individuals found in Figs. 17, 18, 19, 24, 25, and 40, using the screen of Fig. 23 individually and in detail.

While the above description has been made with reference to cases where the horizontal axis coordinate of each of the two true peaks is set to zero when the waveforms of the heterozygous individuals are displayed in an overlapped manner, only one of the left and right true peaks may be shown at zero on the horizontal axis.

In a method for listing the alternative conditions in 1601 in Fig. 16, in addition to listing alternatives designated by the user in advance, self-evidently the alternatives "homozygotes" and "heterozygous individuals in which the two alleles are separated by X units (X=1,2,...)" may be prepared. Further alternatively, a method may be employed whereby the peak interval that appear in an allelic ladder is used as the aforementioned X. While Fig. 16 shows the interval of two alleles in terms of units, it may be designated in terms of bases instead of units so as to take into consideration the insertion/deletion polymorphism or the compound marker (in which a plurality of polymorphisms are contained in a PCR amplified fragment).

While the base range in Function 5 has been described to be displayed in accordance with the value designated by the user, as shown in 1603 of Fig. 16, the fragment length between the ends of an allelic ladder, or, if the fragment length range of the particular marker is known, the value between the ends thereof, may be given as an initial value.

Further, while it has been described that the enlargement of the user-designated range in connection with Function 11 is carried out in a range designated by the user using the pointing device 603, as indicated by 3900 in Fig. 39, the same fragment length range may be designated by the user using a keyboard 602.

Furthermore, while the foregoing description has been made with reference to cases where PCR and electrophoresis are carried out as experiments to extract and detect sites where a microsatellite appears on the genome, the same applies to cases involving other experiments (such as mass analysis for detection instead of electrophoresis). The same is also true with DNA markers other than microsatellites, or polymorphism marker other than nucleic DNA, such as mitochondria, or biomarkers.

While the method and apparatus for displaying gene information according to the invention have been described with reference to specific embodiments, the invention is not limited to such embodiments.

The method and apparatus for displaying gene information according to the invention are realized by an OS, applications, databases, and so on that are constructed on hardware resources including a computer CPU, a memory, an auxiliary storage device, a display unit, an input device, and so on. A DNA fragment is subjected to PCR amplification and electrophoresis, and the resultant waveform data obtained by fluorescence analysis and the result of automatic determination of such data are displayed to the user in an easily understandable manner. Such information processing is carried out using the aforementioned hardware resources in specific ways. Thus, the invention corresponds to a technical concept utilizing a law of nature, and it can be utilized in various industries related to medicine, biology, and so on.

## Claims

1. A gene information display apparatus for displaying the result of analysis of the length of a DNA fragment based on a detection signal obtained from a PCR amplification product thereof, thereby assisting a user in identifying individuals with erroneous genotype determination, comprising:
a processing unit for processing detection signals from the PCR amplification products of a plurality of individuals for the same marker so that the detection signals are displayed
in an overlapped manner on a display, wherein the intensity of the detection signal is shown on a first axis and the fragment length is shown on a second axis, wherein the result of analysis contains information indicating one or two true peaks in the detection signal from the PCR amplification product that are judged to be indicative of the length of the DNA fragment, **characterized in that** the processing unit groups the individuals according to the number of true peaks in the detection signal of the PCR amplification product of each individual and the distance between two true peaks, and then displays the detection signals from the PCR amplification products in one of the groups in an overlapped manner in a graph,
wherein the processing unit standardizes the amplification product of each individual prior to displaying it with respect to the fragment length of a true peak in each PCR amplification product, by translating the individual signals along the second axis so that the position of the peaks that have been determined to be true peaks in each signal are aligned, thereby allowing a user to identify individuals having signals displayed differently from those of other individuals as possibly erroneously genotyped.

2. The gene information display apparatus according to claim 1, wherein the processing unit standardizes the detection signal intensity with respect to the detection signal intensity of a true peak or a highest peak in the signal in each PCR amplification product by multiplying the signal intensities of each signal by a constant number so that the heights of the highest peak or of said true peak in each of the signals are equalized.

3. The gene information display apparatus according to claim 1, wherein the processing unit displays a detection signal from a PCR amplification product that contains two true peaks after performing a correction to equalize the signal intensities of the two true peaks.

4. The gene information display apparatus according to claim 1, wherein the processing unit displays, in a graph in an overlapped manner, the detection signals from the PCR amplification products of only those individuals of which the length of a DNA fragment indicated by a true peak is included in a preset range of fragment lengths.

5. The gene information display apparatus according to claim 1, wherein the result of analysis includes information identifying a +A peak, the +A peak corresponding to the detection signal of a PCR amplification product to which one adenine is added at the end thereof, and wherein the processing unit displays the detection signal of the PCR amplification product from each individual after performing a correction to eliminate the +A peak therefrom.

6. The gene information display apparatus according to claim 1, wherein the result of analysis includes information identifying a +A peak, the +A peak corresponding to the detection signal of a PCR amplification product to which one adenine is added at the end thereof, and the processing unit displays the +A peak in the detection signal of a PCR amplification product from each individual in a different manner.

7. The gene information display apparatus according to claim 1, wherein the processing unit enlarges or reduces the fragment length intervals of the PCR amplification product between two true peaks or a true peak and a noise peak such that each fragment length interval assumes an integer value.

8. The gene information display apparatus according to claim 1, wherein the processing unit explicitly displays the height of the detection signal of a PCR amplification product of each individual.

9. The gene information display apparatus according to claim 1, wherein the processing unit displays the detection signals of the PCR amplification products from a plurality of individuals in an overlapped manner except for a specific individual.

10. The gene information display apparatus according to claim 1, wherein the processing unit displays a specific fragment length range in an enlarged manner.

11. The gene information display apparatus according to claim 1, wherein the processing unit displays each detection signal in accordance with a mode allocated to each fragment length.

12. A computer implemented method for displaying the result of analysis of the length of a DNA fragment based on a detection signal from a PCR amplification product for assisting a user in identifying individuals with erroneous genotype determination, the method comprising the step of:
displaying in a graph the detection signals from the PCR amplification products of a plurality of individuals for the same marker in an overlapped manner, wherein the intensity of the detection signal is shown on a first axis and the fragment length is shown on a second axis, wherein the result of analysis includes information indicating one or two true peaks in the detection signal from the PCR amplification product that are judged to be indicative of the length of the DNA fragment, **characterized in that** the method comprises the steps of:
grouping the individuals according to the number of true peaks in a detection signal from the PCR amplification product of each individual and the distance between two true peaks, and displaying in a graph the detection signals from the PCR amplification products in one of the groups in an overlapped manner, wherein the PCR amplification product from each individual is standardized prior to displaying it with respect to the fragment length of a true peak in each PCR amplification product, by translating the individual signals along the second axis so that the position of the peaks that have been determined to be true peaks in each signal are aligned, thereby allowing a user to identify individuals having signals displayed differently from those of other individuals as possibly erroneously genotyped.

13. The method for displaying gene information according to claim 12,
wherein the detection signal intensity of the PCR amplification product from each individual is standardized with respect to the detection signal intensity of a true peak or the highest peak in each PCR amplification product by multiplying the signal intensities of each signal by a constant number so that the heights of the highest peak or of said true peak in each of the signals are equalized.

14. The method for displaying gene information according to claim 12, the method comprising the step of:
displaying a detection signal from a PCR amplification product that contains two true peaks after performing a correction to equalize the signal intensity of the two true peaks.

15. The method for displaying gene information according to claim 12, the method comprising the step of:
displaying, in a graph in an overlapped manner, the detection signals from the PCR amplification products of only those individuals of which the length of a DNA fragment indicated by a true peak is included in a preset range of fragment lengths.

16. The method for displaying gene information according to claim 12, wherein the result of analysis includes information identifying a +A peak, the +A peak corresponding to the detection signal of the PCR amplification product to which one adenine is added at the end thereof, wherein the detection signal from the PCR amplification product of each individual is displayed after performing a correction to eliminate the +A peak therefrom,

17. The method for displaying gene information according to claim 12, wherein the result of analysis includes information identifying a +A peak, the +A peak corresponding to the detection signal of a PCR amplification product to which one adenine is added at the end thereof, and wherein the +A peak in the detection signal from the PCR amplification product of each individual is displayed in a different manner.

18. The method for displaying gene information according to claim 12, wherein the fragment length intervals in the PCR amplification product between two true peaks or a true peak and a noise peak are enlarged or reduced such that each frequency length interval assumes an integer value.

19. The method for displaying gene information according to claim 12, wherein the height of the detection signal from the PCR amplification product of each individual is explicitly displayed.

20. The method for displaying gene information according to claim 12, wherein the detection signals from the PCR amplification products of a plurality of individuals are displayed in an overlapped manner except for a specific individual.

21. The method for displaying gene information according to claim 12, wherein only a specific fragment length range is enlarged.

22. The method for displaying gene information according to claim 12, wherein each detection signal is displayed in accordance with a mode allocated to each fragment length.

23. A computer program adapted to carry out the method for displaying gene information according to any one of claims 12 to 22 in a computer system equipped with a processing unit and a display unit.

## Patentansprüche

1. Geninformation-Anzeigevorrichtung zum Anzeigen des Ergebnisses der Analyse der Länge eines DNA-Fragments basierend auf einem Detektionssignal, welches von einem PCR-Verstärkungsprodukt desselben erhalten wird, um einem Nutzer dabei zu helfen, Individuen mit einer fehlerhaften Genotyp-Bestimmung zu identifizieren, die Folgendes umfasst:
eine Verarbeitungseinheit zum Verarbeiten von Detektionssignalen von den PCR-Verstärkungsprodukten einer Mehrzahl von Individuen für denselben Marker, derart, dass die Detektionssignale auf überlappende Weise auf einer Anzeige angezeigt werden, wobei die Intensität des Detektionssignals auf einer ersten Achse gezeigt wird und die Fragmentlänge auf einer zweiten Achse gezeigt wird, wobei das Ergebnis der Analyse Information enthält, die eine oder zwei wahre Spitzen in dem Detektionssignal des PCR-Verstärkungsprodukts anzeigt, bezüglich derer entschieden wurde, dass sie indikativ für die Länge des DNA-Fragments sind, **dadurch gekennzeichnet, dass** die Verarbeitungseinheit die Individuen nach der Anzahl von wahren Spitzen in dem Detektionssignal des PCR-Verstärkungsprodukts eines jeden Individuums und
nach dem Abstand zwischen zwei wahren Spitzen gruppiert und dann die Detektionssignale der PCR-Verstärkungsprodukte in einer der Gruppen überlappend in einem Graph anzeigt,
wobei die Prozesseinheit das Verstärkungsprodukt eines jeden Individuums vor dem Anzeigen bezüglich der Fragmentlänge einer wahren Spitze in einem jeden PCR-Verstärkungsprodukt standardisiert, indem die individuellen Signale entlang der zweiten Achse so verschoben werden, dass die Position der Spitzen, die als wahre Spitzen in einem jeden Signal ermittelt wurden, miteinander ausgerichtet sind, wodurch es einem Nutzer gestattet wird, Individuen mit Signalen, die anders als solche von anderen Individuen angezeigt werden, als möglicherweise falsch genotypisiert zu identifizieren.

2. Geninformation-Anzeigevorrichtung nach Anspruch 1, bei der die Verarbeitungseinheit die Detektionssignal-Intensität bezüglich der Detektionssignal-Intensität einer wahren Spitze oder einer höchsten Spitze in dem Signal in einem jeden PCR-Verstärkungsprodukt standardisiert, indem sie die Signalintensitäten eines jeden Signals mit einer konstanten Zahl multipliziert, so dass die Höhen der höchsten Spitze oder der wahren Spitze in einem jeden Signal angeglichen werden.

3. Geninformation-Anzeigevorrichtung nach Anspruch 1, bei der die Verarbeitungseinheit ein Detektionssignal eines PCR-Verstärkungsprodukts, welches zwei wahre Spitzen enthält, anzeigt, nachdem eine Korrektur durchgeführt wurde, um die Intensitäten der beiden wahren Spitzen anzugleichen.

4. Geninformation-Anzeigevorrichtung nach Anspruch 1, bei der die Verarbeitungseinheit die Detektionssignale der PCR-Verstärkungsprodukte nur solcher Individuen in einem Graph auf überlappende Weise anzeigt, deren DNA-Fragmentlänge, wie sie durch eine wahre Spitze repräsentiert ist, in einem vorbestimmten Bereich von Fragmentlängen enthalten ist.

5. Geninformation-Anzeigevorrichtung nach Anspruch 1, bei der das Ergebnis der Analyse Information enthält, die eine +A-Spitze identifiziert, wobei die +A-Spitze dem Detektionssignal eines PCR-Verstärkungsprodukts entspricht, zu dem ein Adenin an dessen Ende zugefügt ist, und wobei die Verarbeitungseinheit das Detektionssignal des PCR-Verstärkungsprodukts eines jeden Individuums anzeigt, nachdem eine Korrektur durchgeführt wurde, um die +A-Spitze daraus zu eliminieren.

6. Geninformation-Anzeigevorrichtung nach Anspruch 1, bei der das Ergebnis der Analyse Information enthält, die eine +A-Spitze identifiziert, wobei die +A-Spitze dem Detektionssignal eines PCR-Verstärkungsprodukts entspricht, zu dem an dessen Ende ein Adenin zugefügt ist, und wobei die Verarbeitungseinheit die +A-Spitze in dem Detektionssignal eines PCR-Verstärkungsprodukts von einem jeden Individuum auf unterschiedliche Weise anzeigt.

7. Geninformation-Anzeigevorrichtung nach Anspruch 1, bei der die Verarbeitungseinheit die Fragmentlängen-Intervalle des PCR-Verstärkungsprodukts zwischen zwei wahren Spitzen oder einer wahren Spitze und einer Rausch-Spitze so vergrößert oder verringert, dass ein jedes Fragmentlängen-Intervall einen ganzzahligen Wert annimmt.

8. Geninformation-Anzeigevorrichtung nach Anspruch 1, bei der die Verarbeitungseinheit die Höhe des Detektionssignals eines PCR-Verstärkungsprodukts für ein jedes Individuum explizit anzeigt.

9. Geninformation-Anzeigevorrichtung nach Anspruch 1, bei der die Verarbeitungseinheit die Detektionssignale der PCR-Verstärkungsprodukte von einer Mehrzahl von Individuen auf überlappende Weise außer für ein spezifisches Individuum anzeigt.

10. Geninformation-Anzeigevorrichtung nach Anspruch 1, bei der die Verarbeitungseinheit eine spezifische Fragmentlänge auf vergrößerte Weise anzeigt.

11. Geninformation-Anzeigevornchtung nach Anspruch 1, bei der die Verarbeitungseinheit ein jedes Detektionssignal gemäß einem Modus anzeigt, der für eine jede Fragmentlänge zugewiesen wird.

12. Computerimplementiertes Verfahren zum Anzeigen des Ergebnisses der Analyse der Länge eines DNA-Fragments basierend auf einem Detektionssignal aus einem PCR-Verstärkungsprodukt, um einem Nutzer zu helfen, Individuen mit falscher Genotypbestimmung zu identifizieren, wobei das Verfahren die folgenden Schritte umfasst:
Anzeigen der Detektionssignale der PCR-Verstärkungsprodukte einer Mehrzahl von Individuen für denselben Marker auf überlappende Weise in einem Graph, wobei die Intensität des Detektionssignals auf einer ersten Achse gezeigt wird und die Fragmentlänge auf einer zweiten Achse gezeigt wird, wobei das Ergebnis der Analyse Information enthält, die eine oder zwei wahre Spitzen in dem Detektionssignal des PCR-Verstärkungsprodukts anzeigt, bezüglich derer entschieden wurde, dass sie indikativ für die Länge des DNA-Fragments sind, **dadurch gekennzeichnet, dass** das Verfahren die folgenden Schritte umfasst:
Gruppieren der Individuen gemäß der Anzahl von wahren Spitzen in einem Detektionssignal des PCR-Verstärkungsprodukts eines jeden Individuums und gemäß des Abstandes zwischen zwei wahren Spitzen, und Anzeigen der Detektionssignale der PCR-Verstärkungsprodukte in einer der Gruppen auf überlappende Weise in einem Graph,
wobei das PCR-Verstärkungsprodukt eines jeden Individuums vor dem Anzeigen bezüglich der Fragmentlänge einer wahren Spitze in einem jeden PCR-Verstärkungsprodukt standardisiert wird, indem die individuellen Signale entlang der zweiten Achse so verschoben werden, dass die Position der Spitzen, die als wahre Spitzen in einem jeden Signal ermittelt wurden, miteinander ausgerichtet sind, wodurch es einem Nutzer gestattet wird, Individuen mit Signalen, die anders als solche von anderen Individuen dargestellt sind, als möglicherweise falsch genotypisiert zu identifizieren.

13. Verfahren zum Anzeigen von Geninformation nach Anspruch 12, wobei die Detektionssignalintensität des PCR-Verstärkungsprodukts eines jeden Individuums bezüglich der Detektionssignalintensität einer wahren Spitze oder der höchsten Spitze in einem jeden PCR-Verstärkungsprodukt standardisiert wird, indem diese Signalintensitäten eines jeden Signals mit einer konstanten Zahl multipliziert werden, so dass die Höhen der höchsten Spitze oder der wahren Spitze in einem jeden der Signale angeglichen werden.

14. Verfahren zum Anzeigen von Geninformation nach Anspruch 12, wobei das Verfahren den folgenden Schritt umfasst:
Anzeigen eines Detektionssignals eines PCR-Verstärkungsprodukts, welches zwei wahre Spitzen enthält, nach Durchführen einer Korrektur, um die Signalintensität der zwei wahren Spitzen anzugleichen.

15. Verfahren zum Anzeigen von Geninformation nach Anspruch 12, wobei das Verfahren den folgenden Schritt umfasst:
Anzeigen der Detektionssignale der PCT-Verstärkungsprodukte nur solcher Individuen, deren DNA-Fragmentlänge, wie sie durch eine wahre Spitze angezeigt wird, in einem vorbestimmten Fragmentlängenbereich liegt, in einem Graph auf überlappende Weise.

16. Verfahren zum Anzeigen von Geninformation nach Anspruch 12, bei dem das Ergebnis der Analyse Information enthält, die eine +A-Spitze identifiziert, wobei die +A-Spitze dem Detektektionssignal des PCR-Verstärkungsprodukts entspricht, zu dem an einem Ende ein Adenin hinzugefügt wurde, wobei das Detektionssignal des PCR-Verstärkungsprodukts eines jeden Individuums angezeigt wird, nachdem eine Korrektur durchgeführt wurde, um die +A-Spitze daraus zu eliminieren.

17. Verfahren zum Anzeigen von Geninformation nach Anspruch 12, bei dem das Ergebnis der Analyse Information enthält, die eine +A-Spitze identifiziert, wobei die +A-Spitze dem Detektionssignal eines PCR-Verstärkungsprodukts entspricht, zu dem an einem Ende ein Adenin zugefügt ist, und wobei die +A-Spitze in dem Detektionssignal des PCR-Verstärkungsprodukts eines jeden Individuums auf eine unterschiedliche Weise dargestellt wird.

18. Verfahren zum Anzeigen von Geninformation nach Anspruch 12, bei dem die Fragmentlängen-Intervalle in dem PCR-Verstärkungsprodukt zwischen zwei wahren Spitzen oder einer wahren Spitze und einer Rausch-Spitze so vergrößert oder verringert werden, dass ein jedes Frequenzlängenintervall einen ganzzahligen Wert annimmt.

19. Verfahren zum Anzeigen von Geninformation nach Anspruch 12, bei dem die Höhe des Detektionssignals des PCR-Verstärkungsprodukts eines jeden Individuums explizit angezeigt wird.

20. Verfahren zum Anzeigen von Geninformation nach Anspruch 12, bei dem die Detektionssignale der PCR-Verstärkungsprodukte einer Mehrzahl von Individuen auf überlappende Weise dargestellt werden, außer für ein spezifisches Individuum.

21. Verfahren zum Anzeigen von Geninformation nach Anspruch 12, bei dem nur ein spezifischer Fragmentlängenbereich vergrößert wird.

22. Verfahren zum Anzeigen von Geninformation nach Anspruch 12, bei dem ein jedes Detektionssignal gemäß einem Modus angezeigt wird, welcher für eine jede Fragmentlänge zugewiesen wird.

23. Computerprogramm, welches geeignet ist, das Verfahren zum Anzeigen von Geninformation gemäß einem der Ansprüche 12 - 22 in einem Computersystem auszuführen, welches mit einer Verarbeitungseinheit und einer Anzeigeeinheit versehen ist.

## Revendications

1. Dispositif d'affichage d'informations génétiques pour afficher les résultats d'une analyse de longueur d'un fragment d'ADN sur la base d'un signal de détection obtenu à partir d'un produit d'amplification PCR de celui-ci, assistant ainsi un utilisateur dans l'identification d'individus ayant une détermination de génotype erronée, comprenant :
une unité de traitement pour traiter des signaux de détection provenant des produits d'amplification PCR d'une pluralité d'individus pour le même marqueur de telle sorte que les signaux de détection sont affichés avec un chevauchement sur un afficheur, l'intensité du signal de détection étant représentée sur un premier axe et la longueur du fragment étant représentée sur un deuxième axe, dans lequel le résultat de l'analyse contient des informations indiquant un ou deux pics vrais dans le signal de détection provenant du produit d'amplification PCR qui sont considérés comme étant indicatifs de la longueur du fragment d'ADN, **caractérisé en ce que** l'unité de traitement groupe les individus en fonction du nombre de pics vrais dans le signal de détection du produit d'amplification PCR de chaque individu et de la distance entre deux pics vrais, puis affiche les signaux de détection provenant des produits d'amplification PCR dans un des groupes avec chevauchement dans le graphe,
dans lequel l'unité de traitement standardise le produit d'amplification de chaque individu avant de l'afficher, en ce qui concerne la longueur du fragment d'un pic vrai dans chaque produit d'amplification PCR, en translatant les signaux individuels suivant le deuxième axe de telle sorte que la position des pics qui ont été déterminés comme étant des pics vrais dans chaque signal sont alignés, permettant ainsi à un utilisateur d'identifier des individus ayant des signaux affichés différemment de ceux d'autres individus comme étant éventuellement génotypés de façon erronée.

2. Dispositif d'affichage d'informations génétiques selon la revendication 1, dans lequel l'unité de traitement standardise l'intensité du signal de détection en ce qui concerne l'intensité du signal de détection d'un pic vrai ou du pic le plus élevé dans le signal dans chaque produit d'amplification PCR en multipliant les intensités de chaque signal par un nombre constant de telle sorte que les hauteurs du pic le plus élevé ou du pic vrai dans chacun des signaux sont égalisées.

3. Dispositif d'affichage d'informations génétiques selon la revendication 1, dans lequel l'unité de traitement affiche un signal de détection provenant d'un produit d'amplification PCR qui contient deux pics vrais après avoir effectué une correction pour égaliser les intensités des signaux des deux pics vrais.

4. Dispositif d'affichage d'informations génétiques selon la revendication 1, dans lequel l'unité de traitement affiche, sur un graphique et avec chevauchement, les signaux de détection provenant des produits d'amplification PCR des seuls individus dont la longueur d'un fragment ADN indiquée par un pic vrai est incluse dans une plage présente de longueurs de fragment.

5. Dispositif d'affichage d'informations génétiques selon la revendication 1, dans lequel le résultat de l'analyse comprend des informations identifiant un pic +A, le pic +A correspondant au signal de détection d'un produit d'amplification PCR auquel est ajoutée une adénine à la fin, et dans lequel l'unité de traitement affiche le signal de détection du produit d'amplification PCR provenant de chaque individu après avoir effectué une correction pour éliminer le pic +A de celui-ci.

6. Dispositif d'affichage d'informations génétiques selon la revendication 1, dans lequel le résultat de l'analyse comprend des informations identifiant un pic +A, le pic +A correspondant au signal de détection d'un produit d'amplification PCR auquel est ajoutée une adénine à la fin, et l'unité de traitement affiche le pic +A dans le signal de détection d'un produit d'amplification PCR provenant de chaque individu de façon différente.

7. Dispositif d'affichage d'informations génétiques selon la revendication 1, dans lequel l'unité de traitement agrandit ou diminue les intervalles de longueur de fragment du produit d'amplification PCR entre deux pics vrais ou un pic vrai et un pic de bruit de telle sorte que chaque intervalle de longueur de fragment prend une valeur entière.

8. Dispositif d'affichage d'informations génétiques selon la revendication 1, dans lequel l'unité de traitement affiche de façon explicite la hauteur du signal de détection d'un produit d'amplification PCR de chaque individu.

9. Dispositif d'affichage d'informations génétiques selon la revendication 1, dans lequel l'unité de traitement affiche les signaux de détection des produits d'amplification PCR provenant d'une pluralité d'individus avec un chevauchement excepté pour un individu particulier.

10. Dispositif d'affichage d'informations génétiques selon la revendication 1, dans lequel l'unité de traitement affiche une plage particulière de longueurs de fragment de façon agrandie.

11. Dispositif d'affichage d'informations génétiques selon la revendication 1, dans lequel l'unité de traitement affiche chaque signal de détection conformément à un mode alloué à chaque longueur de fragment.

12. Procédé mis en oeuvre sur ordinateur pour afficher les résultats d'une analyse de longueur d'un fragment d'ADN sur la base d'un signal de détection provenant d'un produit d'amplification PCR pour assister un utilisateur dans l'identification d'individus ayant une détermination de génotype erronée, le procédé comprenant les étapes suivantes :
afficher dans un graphique les signaux de détection provenant de produits d'amplification PCR d'une pluralité d'individus pour le même marqueur avec un chevauchement, l'intensité du signal de détection étant représentée sur un premier axe et la longueur de fragment étant représentée sur un deuxième axe, dans lequel les résultats de l'analyse comprennent des informations indiquant un ou deux pics vrais dans le signal de détection provenant du produit d'amplification PCR qui sont considérés comme étant indicatifs de la longueur du fragment d'ADN, **caractérisé en ce que** le procédé comprend les étapes suivantes :
grouper les individus en fonction du nombre de pics vrais dans un signal de détection provenant du produit d'amplification PCR de chaque individu et de la distance entre deux pics vrais, et afficher dans un graphique les signaux de détection provenant de produits d'amplification PCR dans un des groupes avec un chevauchement, dans lequel le produit d'amplification PCR provenant de chaque individu est standardisé avant affichage en ce qui concerne la longueur de fragment d'un pic vrai dans chaque produit d'amplification PCR, en translatant les signaux individuels suivant le deuxième axe de telle sorte que la position des pics qui ont été déterminés comme étant des pics vrais dans chaque signal sont alignés, permettant ainsi à l'utilisateur d'identifier des individus ayant des signaux affichés de façon différente de ceux d'autres individus comme étant éventuellement génotypés de façon erronée.

13. Procédé d'affichage d'informations génétiques selon la revendication 12,
dans lequel l'intensité du signal de détection du produit d'amplification PCR provenant de chaque individu est standardisée en ce qui concerne l'intensité du signal de détection d'un pic vrai ou du pic le plus haut dans chaque produit d'amplification PCR en multipliant les intensités de chaque signal par un nombre constant de telle sorte que les hauteurs du pic le plus élevé ou du pic vrai dans chacun des signaux sont égalisées.

14. Procédé d'affichage d'informations génétiques selon la revendication 12, ce procédé comprenant l'étape suivante :
afficher un signal de détection provenant d'un produit d'amplification PCR qui contient deux pics vrais après avoir effectué une correction pour égaliser l'intensité de signal des deux pics vrais.

15. Procédé d'affichage d'informations génétiques selon la revendication 12, le procédé comprenant l'étape suivante :
afficher, dans un graphique et avec chevauchement, les signaux de détection provenant des produits d'amplification PCR des seuls individus dont la longueur de fragment d'ADN indiquée par un pic vrai est incluse dans une plage prédéterminée de longueurs de fragment.

16. Procédé d'affichage d'informations génétiques selon la revendication 12, dans lequel le résultat de l'analyse comprend des informations identifiant un pic +A, le pic +A correspondant au signal de détection du produit d'amplification PCR auquel est ajoutée une adénine à la fin, dans lequel le signal de détection provenant du produit d'amplification PCR de chaque individu est affiché après avoir effectué une correction pour éliminer le pic +A de celui-ci.

17. Procédé d'affichage d'informations génétiques selon la revendication 12, dans lequel le résultat de l'analyse comprend des informations identifiant un pic +A, le pic +A correspondant au signal de détection d'un produit d'amplification PCR auquel est ajoutée une adénine à la fin, et dans lequel le pic +A dans le signal de détection provenant du produit d'amplification PCR de chaque individu est affiché d'une manière différente.

18. Procédé d'affichage d'informations génétiques selon la revendication 12, dans lequel les intervalles de longueur de fragment dans le produit d'amplification PCR entre deux pics vrais ou un pic vrai et un pic de bruit sont agrandis ou diminués de telle sorte que chaque intervalle de longueur de fréquence prend une valeur entière.

19. Procédé d'affichage d'informations génétiques selon la revendication 12, dans lequel la hauteur du signal de détection provenant du produit d'amplification PCR de chaque individu est affichée de façon explicite.

20. Procédé d'affichage d'informations génétiques selon la revendication 12, dans lequel les signaux de détection provenant des produits d'amplification PCR d'une pluralité d'individus sont affichés avec un chevauchement excepté pour un individu particulier.

21. Procédé d'affichage d'informations génétiques selon la revendication 12, dans lequel seule une plage de longueurs de fragment particulière est agrandie.

22. Procédé d'affichage d'informations génétiques selon la revendication 12, dans lequel chaque signal de détection est affiché conformément à un mode alloué à chaque longueur de fragment.

23. Programme d'ordinateur apte à réaliser le procédé d'affichage d'informations génétiques selon l'une quelconque des revendications 12 à 22 dans un système d'ordinateur équipé d'une unité de traitement et d'une unité d'affichage.
